# EUROPEAN PATENT APPLICATION

(11) **EP 2 011 877 A2**
(43) Date of publication of application: **07.01.2009**
(21) Application number: 08011334.3
(22) Date of filing: 25.07.2002
(51) Int. Cl.: C12N 15/62, C12N 15/63, C07K 14/47, C07K 14/705, C07K 19/00, A61K 38/17, A61K 39/02

(54) **Conjugates for the modulation of immune responses**

(30) Priority: 25.07.2001 GB 0118155
(62) Divisional of application: 02745675.5
(71) Applicant: Celldex Therapeutics Limited, Cambridge CB4 0PE (GB)
(72) Inventor: Bodmer, Mark William c/o Celldex Therapeutics Ltd., Cambridge, CB4 0EY (GB); Champion, Brian Robert c/o Celldex Therapeutics Ltd., Cambridge, CB4 0EY (GB); Mckenzie, Grahame James c/o Celldex Therapeutics Ltd., Cambridge, CB4 0EY (GB); Nye, Lucy Emma c/o Celldex Therapeutics Ltd., Cambridge, CB4 0EY (GB)
(74) Representative: Mallalieu, Catherine Louise

(57) **Abstract**

A conjugate comprising first and second sequences wherein the first sequence is an antibody or antibody fragment which is capable of binding to an APC surface molecule, or a polynucleotide encoding therefor, and the second sequence comprises a polypeptide for Notch signalling transduction or a polynucleotide sequence encoding said polypeptide for Notch signalling transduction.

## Description

### Field of the Invention

The present invention relates to a molecule and method for modifying T cell activation via targeting of a protein for T cell signalling modulation, and particularly, but not exclusively, a protein for Notch signalling modulation.

### Background of the Invention

Immunological tolerance to self-antigens is vital to the proper functioning of the mammalian immune system. In addition to the deletion of self-reacting T cells in the thymus, active suppression mediated by regulatory T cells has recently been identified as an important mechanism for maintaining peripheral tolerance (WO98/20142). In autoimmune diseases such as multiple sclerosis, rheumatoid arthritis or diabetes, there is a failure of the proper regulation of tolerance. Improved treatment methods for reestablishing tolerance are desirable for autoimmune diseases. Similarly in allergic conditions and for transplantation of an organ or tissue from a donor individual, induction of tolerance to particular foreign antigens or profiles of foreign antigens is desirable.

It has recently been shown that it is possible to generate a class of regulatory T cells which are able to transmit antigen-specific tolerance to other T cells, a process termed infectious tolerance (WO98/20142). The functional activity of these cells can be mimicked by over-expression of a Notch ligand protein on their cell surfaces or on the surface of antigen presenting cells. In particular, regulatory T cells can be generated by over-expression of a member of the Delta or Serrate family of Notch ligand proteins. Delta or Serrate induced T cells specific to one antigenic epitope are also able to transfer tolerance to T cells recognising other epitopes on the same or related antigens, a phenomenon termed "epitope spreading" (Hoyne et al).

In addition, WO00/36089 describes a method for producing a lymphocyte or antigen presenting cell (APC) having tolerance to an allergen or antigen which method comprises incubating a lymphocyte or APC obtained from a human or animal patient with (i) a composition capable of upregulating expression of an endogenous Notch or Notch ligand in the lymphocyte and/or APC and (ii) the allergen or antigen.

Notch ligand expression also plays a role in cancer. Indeed, upregulated Notch ligand expression has been observed in some tumour cells. These tumour cells are capable of rendering T cells unresponsive to restimulation with a specific antigen, thus providing a possible explanation of how tumour cells prevent normal T cell responses. By downregulating Notch signalling *in vivo* in T cells, it may be possible to prevent tumour cells from inducing immunotolerance in those T cells that recognise tumour specific antigens. In turn, this would allow the T cells to mount an immune response against the tumour cells (WO00/135990).

A description of the Notch signalling pathway and conditions affected by it may be found in our published PCT Applications WO 98/20142, WO 00/36089 and WO 0135990. The text of each of PCT/GB97/03058 (WO 98/20142), PCT/GB99/04233 (WO 00/36089) and PCT/GB00/04391 (WO 0135990) is hereby incorporated herein by reference

However, there remains a need in the art for the provision of further diagnostic or therapeutic compositions useful in the detection, prevention and treatment of T cell mediated diseases or disorders.

### Summary of the Invention

The present invention addresses the above mentioned problems by delivering an effective Notch signal directly to T cells. More generally the present invention relates to the concept of delivering a modulator of T cell signalling, such as a Notch ligand, to an antigen presenting cell (APC). The targeting approach disclosed uses, for example, the major histocompatibility complex (MHC) class II binding motif from a superantigen coupled to a modulator of the Notch signalling pathway. Superantigens bind both MHC class II molecules and subsets of T cell receptors and thus effectively cross-link APCs to T cells and activate cells polyclonally. The molecular regions of these molecules that impart T cell receptor (TCR) and MHC class II binding have been defined structurally and have been shown to be distinct regions of the molecule. By using the MHC class II binding domain with a modulator of the Notch signalling pathway we can focus the activity of the Notch signalling pathway modulator to the APCs at the site of delivery. Further, the domain lacks toxin activity because it cannot find the T cell receptor to activate T cells.

### Statements of the Invention

According to one aspect of the present invention there is provided a conjugate comprising a first and a second sequence wherein the first sequence comprises a polypeptide which is capable of binding to an APC, or a polynucleotide encoding therefor, and the second sequence comprises a polypeptide comprising a modulator of a signalling pathway in a T cell or a polynucleotide encoding therefor.

For ease of reference the sequence which is capable of binding to an APC is sometimes referred to as a targeting sequence, targeting molecule or target protein.

Thus, the present invention relates to a conjugate which is a molecule comprising at least one targeting protein linked to at least one protein for T cell signalling modulation or polynucleotide encoding said T cell ligand protein formed through genetic fusion or chemical coupling. By "linked" we mean that the first and second sequences are associated such that the second sequence is able to be associated by the first sequence into an APC, i.e. the target cell. Thus, conjugates include fusion proteins in which the targeting protein is linked to a protein for T cell signalling modulation via their polypeptide backbones through genetic expression of a DNA molecule encoding these proteins, directly synthesised proteins and coupled proteins in which pre-formed sequences are associated by a cross-linking agent The term is also used herein to include associations, such as aggregates, of the protein for T cell signalling modulation with the target protein. According to one embodiment the second sequence may comprise a polynucleotide sequence, e.g, a nucleic acid binding domain. This embodiment may be seen as a protein/nucleic acid complex.

By APC we mean a cell expressing MHC class II molecules and able to present antigen to CD4+ T cells. Examples of APCs include macrophages, dentritic cells, B cells, Langerhans cells and virtually any other cell type capable of expressing MHC class II molecules may function as antigen presenting cells (APCs) and are all included in the present invention.

The second sequence may be from the same species as the first sequence, but is present in the conjugate of the invention in a manner different from the natural situation, or from a different species.

The conjugates of the present invention are capable of being bond by or taken up by a population of APCs so that an effector function corresponding to the second polypeptide sequence coupled can take place within a T cell to which the APC presents.

The second sequence of the present invention is a protein which is for T cell signalling modulation. T cell signalling modulation involves transduction, activation or inhibition of the T cell signalling pathways including upstream and downstream events.

The term "modulate" as used herein refers to a change or alteration in the biological activity of the T cell signalling pathway or a target signalling pathway thereof The term "modulator" may refer to antagonists or inhibitors of T cell signalling, i.e. compounds which block, at least to some extent, the normal biological activity of the T cell signalling pathway. Conveniently such compounds may be referred to herein as inhibitors or antagonists. Alternatively, the term "modulator" may refer to agonists of T cell signalling, i.e. compounds which stimulate or upregulate, at least to some extent, the normal biological activity of the T cell signalling pathway. Conveniently such compounds may be referred to as upregulators or agonists.

The modulator of the present invention or indeed the targeting molecule may be an organic compound or other chemical. In one embodiment, the modulator or targeting sequence will be an organic compound composing two or more hydrocarbyl groups.

Here, the term "hydrocarbyl group" means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked *via* a suitable element or group. Thus, the hydrocarbyl group may contain hetero atoms. Suitable hetero atoms will be apparent to those skilled in the art and include, for instance, sulphur, nitrogen and oxygen. The candidate modulator may compose at least one cyclic group. The cyclic group may be a polycyclic group, such as a non-fused polycyclic group. For some applications, the agent comprises at least the one of said cyclic groups linked to another hydrocarbyl group.

In one preferred embodiment, the modulator and/or targeting molecule will be an amino acid sequence or a chemical derivative thereof, or a combination thereof. In another preferred embodiment, the modulator and/or targeting molecule will be a nucleotide sequence - which may be a sense sequence or an anti-sense sequence. The modulator and/or targeting molecule may also be an antibody.

The term "antibody" includes intact molecules as well as fragments thereof, such as Fab, F(ab')2, Fv and scFv which are capable of binding the epitopic determinant. These antibody fragments retain some ability to selectively bind with its antigen or receptor and include, for example:
(i) Fab, the fragment which contains a monovalent antigen-binding fragment of an antibody molecule can be produced by digestion of whole antibody with the enzyme papain to yield an intact light chain and a portion of one heavy chain;
(ii) Fab', the fragment of an antibody molecule can be obtained by treating whole antibody with pepsin, followed by reduction, to yield an intact light chain and a portion of the heavy chain; two Fab' fragments are obtained per antibody molecule;
(iii) F(ab')₂, the fragment of the antibody that can be obtained by treating whole antibody with the enzyme pepsin without subsequent reduction; F(ab')₂ is a dimer of two Fab' fragments held together by two disulfide bonds;
(iv) scFv, including a genetically engineered fragment containing the variable region of a heavy and a light chain as a fused single chain molecule.

General methods of making these fragments are known in the art (See for example, Harlow and Lane, Antibodies: A Laboratory Manual, Cold Spring Harbor Laboratory, New York (1988), which is incorporated herein by reference).

Modulators may be synthetic compounds or natural isolated compounds.

It has been established that T cell activation not only requires a signal through a T cell receptor but may also also a second signal generated by the interaction of costimulatory molecules (also known as accessory molecules), e.g. CD80 and CD86, on the surface of APCs and on T cells, e.g. CD28 or CTLA-4, respectively. By T cell signalling pathway we include not only signalling through the T cell receptor, but also signalling through T cell costimulatory molecules.

Examples of such T cell costimulatory molecules and which may be modulated using the present invention include: Notch, CD28, CTLA4, ICOS, CD40L, CD30, CD27, PD1, CD134, CD137 and TRANCE, but any T cell costimulatory molecule known or subsequently discovered may be employed in the present invention. Preferably the modulator of the T cell signalling pathway comprises the corresponding ligand of such T cell costimulatory molecules. Such ligands are often referred to as APC costimulatory molecules. Any such pairing which is known or becomes available may be employed in the present invention. Examples of such pairings are given below:

| **T cell** | **APC** |
|---|---|
| CD2 | LFA-3(CD58) |
| LFA-1 (CD11a/CD18) | ICAM-1 (CD54) |
| CD28 | B7.1(CD80) |
| CTLA-4 | B7.2 (CD86) |
| CD5 | CD72 |
| CD45 | CD22 |

In a preferred embodiment the present invention is directed to modulation of the Notch signalling pathway and the modulator of a T cell signalling pathway is a protein for Notch signalling transduction.

By a protein which is for Notch signalling transduction we mean a molecule which participates in signalling through Notch receptors including activation of Notch, the downstream events of the Notch signalling pathway, transcriptional regulation of downstream target genes and other non-transcriptional downstream events (e.g. post-translational modification of existing proteins). More particularly, the second sequence is a domain that allows activation of target genes of the Notch signalling pathway, or a polynucleotide sequence which codes therefor.

Key targets for Notch-dependent transcriptional activation are genes of the *Enhancer of split* complex (E[spl]). Moreover these genes have been shown to be direct targets for binding by the Su(H) protein and to be transcriptionally activated in response to Notch signalling. By analogy with EBNA2, a viral coactivator protein that interacts with a mammalian Su(H) homologue CBF1 to convert it from a transcriptional repressor to a transcriptional activator, the Notch intracellular domain, perhaps in association with other proteins may combine with Su(H) to contribute an activation domain that allows Su(H) to activate the transcription of *E(spl)* as well as other target genes. It should also be noted that Su(H) is not required for all Notch-dependent decisions, indicating that Notch mediates some cell fate choices by associating with other DNA-binding transcription factors or be employing other mechanisms to transduce extracellular signals.

According to one aspect of the present invention the second amino acid sequence is Notch or a fragment thereof which retains the signalling transduction ability of Notch or an analogue of Notch which has the signalling transduction ability of Notch.

As used herein the term "analogue of Notch" includes variants thereof which retain the signalling transduction ability of Notch. By "analogue" we include a protein which has Notch signalling transduction ability, but generally has a different evolutionary origin to Notch. Analogues of Notch include proteins from the Epstein Barr virus (EBV), such as EBNA2, BARF0 or LMP2A.

By a protein which is for Notch signalling activation we mean a molecule which is capable of activating Notch, the Notch signalling pathway or any one or more of the components of the Notch signalling pathway.

In a particular embodiment, the molecule will be capable of inducing or increasing Notch or Notch ligand expression. Such a molecule may be a nucleic acid sequence capable of inducing or increasing Notch or Notch ligand expression.

In one embodiment, the molecule will be capable of upregulating expression of the endogenous genes encoding Notch or Notch ligands in target cells. In particular, the molecule may be an immunosuppressive cytokine capable of upregulating the expression of endogenous Notch or Notch ligands in target cells, or a polynucleotide which encodes such a cytokine. Immunosuppressive cytokines include IL-4, IL-10, IL-13, TGF-β and SLIP3 (FLT3) ligand.

Preferably, the molecule will be a polypeptide selected from Noggin, Chordin, Follistatin, Xnr3, fibroblast growth factors and derivatives, fragments, variants and homologues thereof, or a polynucleotide encoding any one or more of the above.

In another embodiment, the molecule may be a Notch ligand, or a polynucleotide encoding a Notch ligand. Notch ligands of use in the present invention include endogenous Notch ligands which are typically capable of binding to a Notch receptor polypeptide present in the membrane of a variety of mammalian cells, for example hemapoietic stem cells.

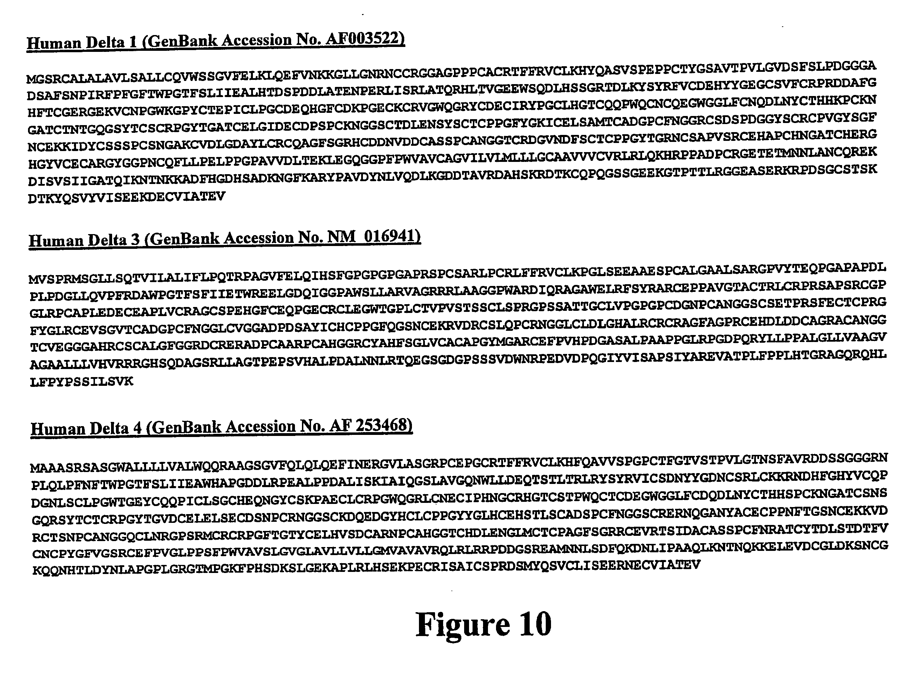

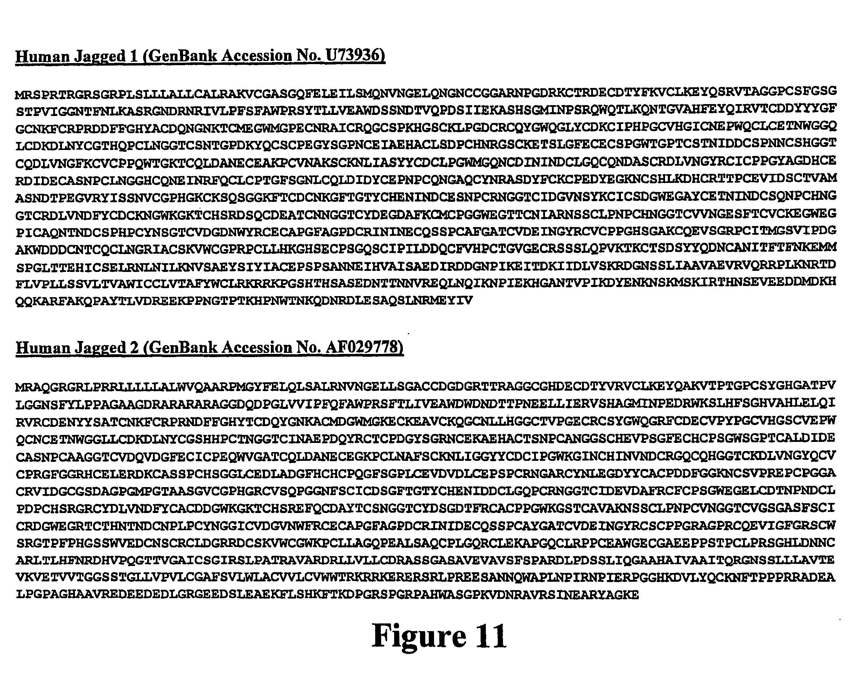

Particular examples of mammalian Notch ligands identified to date include the Delta family, for example Delta or Delta-like 1 (Genbank Accession No. AF003522 - *Homo sapiens),* Delta-3 (Genbank Accession No. AF084576 - *Rattus norvegicus*) and Delta-like 3 *(Mus musculus*) (Genbank Accession No. NM_016941 - *Homo sapiens*) and US 6121045 (Millennium), Delta-4 (Genbank Accession Nos. AB043894 and AF 253468 *- Homo sapiens*) and the Serrate family, for example Serrate-1 and Serrate-2 (WO97/01571, WO96/27610 and WO92/19734), Jagged-1 (Genbank Accession No. U73936 - *Homo sapiens*) and Jagged-2 (Genbank Accession No. AF029778 - *Homo sapiens*)*,* and LAG-2. Homology between family members is extensive. Sequences of human Delta and Jagged ligands are provided in Figure 10 and 11 respectively.

In a preferred embodiment, the activator will be a constitutively active Notch receptor or Notch intracellular domain, or a polynucleotide encoding such a receptor or intracellular domain.

In an alternative embodiment, the activator of Notch signalling will act downstream of the Notch receptor. Thus, for example, the activator of Notch signalling may be a constitutively active Deltex polypeptide or a polynucleotide encoding such a polypeptide. Other downstream components of the Notch signalling pathway of use in the present invention include the polypeptides involved in the Ras/MAPK cascade catalysed by Deltex, polypeptides involved in the proteolytic cleavage of Notch such as Presenilin and polypeptides involved in the transcriptional regulation of Notch target genes, Preferably in a constitutively active form.

By polypeptides for Notch signalling activation is also meant any polypeptides expressed as a result of Notch activation and any polypeptides involved in the expression of such polypeptides, or polynucleotides encoding for such polypeptides.

Activation of Notch signalling may also be achieved by repressing inhibitors of the Notch signalling pathway. As such, polypeptides for Notch signalling activation will include molecules capable of repressing any Notch signalling inhibitors. Preferably the molecule will be a polypeptide, or a polynucleotide encoding such a polypeptide, that decreases or interferes with the production or activity of compounds that are capable of producing an decrease in the expression or activity of Notch, Notch ligands, or any downstream components of the Notch signalling pathway. In a preferred embodiment, the molecules will be capable of repressing polypeptides of the Toll-like receptor protein family, cytokines such as IL-12, IFN-γ, TNF-α, and growth factors such as the bone morphogenetic protein (BMP), BMP receptors and activins, derivatives, fragments, variants and homologues thereof.

By a protein which is for Notch signalling inhibition or a polynucleotide encoding such a protein, we mean a molecule which is capable of inhibiting Notch, the Notch signalling pathway or any one or more of the components of the Notch signalling pathway.

In a particular embodiment, the molecule will be capable of reducing or preventing Notch or Notch ligand expression. Such a molecule may be a nucleic acid sequence capable of reducing or preventing Notch or Notch ligand expression.

Preferably the nucleic acid sequence encodes a polypeptide selected from Toll-like receptor protein family, a cytokine such as IL-12, IFN-γ, TNF-α, or a growth factor such as a bone morphogenetic protein (BMP), a BMP receptor and activins. Preferably the agent is a polypeptide, or a polynucleotide encoding such a polypeptide, that decreases or interferes with the production of compounds that are capable of producing an increase in the expression of Notch ligand, such as Noggin, Chordin, Follistatin, Xnr3, fibroblast growth factors and derivatives, fragments, variants and homologues thereof.

Alternatively, the nucleic acid sequence is an antisense construct derived from a sense nucleotide sequence encoding a polypeptide selected from a Notch ligand and a polypeptide capable of upregulating Notch ligand expression, such as Noggin, Chordin, Follistatin, Xnr3, fibroblast growth factors and derivatives, fragments, variants and homologues thereof

In another preferred embodiment the inhibitor of Notch signalling is a molecule which is capable of modulating Notch-Notch ligand interactions. A molecule may be considered to modulate Notch-Notch ligand interactions if it is capable of inhibiting the interaction of Notch with its ligands, preferably to an extent sufficient to provide therapeutic efficacy.

In this embodiment the molecule may be a polypeptide, or a polynucleotide encoding such a polypeptide, selected from a Toll-like receptor, a cytokine such as IL-12, IFN-γ, TNF-α, or a growth factor such as a BMP, a BMP receptor and activins. Preferably the polypeptide decreases or interferes with the production of an agent that is capable of producing an increase in the expression of Notch ligand, such as Noggin, Chordin, Follistatin, Xnr3, fibroblast growth factors and derivatives, fragments, variants, homologues and analogs thereof.

Preferably when the inhibitor is a receptor or a nucleic acid sequence encoding a receptor, the receptor is activated. Thus, for example, when the agent is a nucleic acid sequence, the receptor is constitutively active when expressed.

Inhibitors of Notch signalling also include downstream inhibitors of the Notch signalling pathway, compounds that prevent expression of Notch target genes or induce expression of genes repressed by the Notch signalling pathway. Examples of such proteins include dominant negative versions of Notch IC, Deltex, Dsh or Numb. Proteins for Notch signalling inhibition will also include variants of the wild-type component of the Notch signalling pathway which have been modified in such a way that their presence blocks rather than transduces the signalling pathway. An example of such a compound would be a Notch receptor which has been modified such that proteolytic cleavage of its intracellular domain is no longer possible.

According to the present invention the first sequence is capable of targeting a second sequence to an APC for presentation to a TCR. In a preferred embodiment the first sequence comprises a polypeptide that is capable of binding to a MHC class II molecule. Preferably the first sequence is or is derived from a superantigen and comprises the MHC class II molecule binding domain thereof. Preferably the first sequence does not include the TCR binding domain of the superantigen.

T cells recognise antigen only in the context of appropriate (i.e. self) MHC molecules. Self MHC is therefore required for effective antigen presentation to T cells, which are activated to offer T cell help or cytotoxic activity. CD4+ T cells, usually T-helper cells, are restricted to recognizing antigen only in association with MHC class II molecules. According to the "associated recognition theory" of antigen and MHC by T cells, a single T cell receptor recognises both MHC and antigen specificities. The T cell receptor (TCR) engages with the antigenic peptide-MHC molecule complex, T cell CD4 molecules bind to a conserved region of the MHC class II molecule.

Thus superantigens generally are certain bacterial and viral glycoproteins that bind TCR and MHC class II antigens outside of the conventional groove for antigenic peptide binding, leading to nonspecific activation of multiple T cell clones.

In another embodiment the first sequence comprises a polypeptide which is capable of binding to another APC surface molecule. Such APC molecules include: CD205 (DEC205), CD204 (Scavenger receptor), CD14, CD206 (Mannose receptor), TLRs, Langerin (CD207), DC-SIGN (CD209), Fcγ receptor 1 (CD64) and Fcγ receptor 2 (CD32), CD68, CD83, CD33, CD54 and BDCA-2,3,4. Other such surface molecules may are known or become available may also be targeted by the first sequence.

It will be appreciated that the first sequence may therefore take the form of an antibody to an APC surface molecule. In a preferred embodiment the antibody is generated against the APC extracellular domain of the APC surface molecule, or a fragment thereof. The production of antibodies is described in for example Kohler and Milstein (1975) Nature 256:495-497.

It will be appreciated that one can apply conventional protein binding assays to identify molecules which bind to APC surface molecules. It will also be appreciated that one can apply structural-based drug design to develop sequences which bind to APC surface molecules.

Any one or more of appropriate targets - such as an amino acid sequence and/or nucleotide sequence - may be used for identifying a compound capable of modulating the T cell signalling pathway and/or a targeting molecule in any of a variety of drug screening techniques. The target employed in such a test may be free in solution, affixed to a solid support, borne on a cell surface, or located intracellularly.

Techniques for drug screening may be based on the method described in Geysen, European Patent No. 0138855, published on September 13, 1984. In summary, large numbers of different small peptide candidate modulators or targeting molecules are synthesized on a solid substrate, such as plastic pins or some other surface. The peptide test compounds are reacted with a suitable target or fragment thereof and washed. Bound entities are then detected - such as by appropriately adapting methods well known in the art. A purified target can also be coated directly onto plates for use in drug screening techniques. Plates of use for high throughput screening (HTS) will be multi-well plates, preferably having 96, 384 or over 384 wells/plate. Cells can also be spread as "lawns". Alternatively, non-neutralising antibodies can be used to capture the peptide and immobilise it on a solid support. High throughput screening, as described above for synthetic compounds, can also be used for identifying organic candidate modulators and targeting molecules.

This invention also contemplates the use of competitive drug screening assays in which neutralising antibodies capable of binding a target specifically compete with a test compound for binding to a target.

Also within the invention are mammalian and microbial host cells comprising such vectors or other polynucleotides encoding the fusion proteins, and their production and use.

A fusion polypeptide as described herein can be targeted to a target population of APCs, by introducing a polynucleotide or other vector encoding the fusion polypeptide into a population of cells, e.g. by transfection or microinjection; expressing the encoding polynucleotide to produce the fusion polypeptide, thereby to cause it to be exported from said population of cells, and to cause it to be taken up by a said APCs.

Alternatively a fusion polypeptide as described herein can be targeted to a target population of APCs, by introducing a polynucleotide or other vector encoding the fusion polypeptide into a first part of the target population of APCs, e.g. by transfection or microinjection; expressing the encoding polymicleotide to produce the fusion polypeptide, thereby to cause it to be exported from said first part of said target population, and to cause it to be taken up by a second part of the target population of cells not directly producing the fusion polypeptide.

Coupled products can also be targeted into a target population of APCs by directly exposing the APCs to a preparation of the coupled products, thereby to cause the target APCs to take them up.

Within the definitions of "proteins" useful in the present invention, the specific amino acid residues may be modified in such a manner that the protein in question retains at least one of its endogenous functions, such modified proteins are referred to as "variants". A variant protein can be modified by addition, deletion and/or substitution of at least one amino acid present in the naturally-occurring protein.

Typically, amino acid substitutions may be made, for example from 1, 2 or 3 to 10 or 20 substitutions provided that the modified sequence retains the required target activity or ability to modulate Notch signalling. Amino acid substitutions may include the use of non-naturally occurring analogues.

The protein used in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity, and/or the amphipathic nature of the residues as long as the target or modulation function is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

For ease of reference, the one and three letter codes for the main naturally occurring amino acids (and their associated codons) are set out below:

| Symbol | 3-letter | Meaning | Codons |
|---|---|---|---|
| A | Ala | Alanine | GCT, GCC, GGA, GCG |
| B | Asp, Asn | Aspartic, | |
| | | Asparagine | GAT,GAC,AAT,AAC |
| C | Cys | Cysteine | TGT,TGC |
| D | Asp | Aspartic | GAT, GAC |
| E | Glu | Glutamic | GAA,GAG |
| F | Phe | Phenylalanine | TTT, TTC |
| G | Gly | Glycine | GGT,GGC,GGA,GGG |
| H | His | Histidine | CAT, CAC |
| I | Ile | Isoleucine | ATT,ATC,ATA |
| K | Lys | Lysine | AAA,AAG |
| L | Leu | Leucine | TTG,TTA,CTT,CTC,CTA,CTG |
| M | Met | Methionine | ATG |
| N | Asn | Asparagine | AAT,AAC |
| P | Pro | Proline | CCT,CCC,CCA,CCG |
| Q | Gln | Glutamine | CAA,CAG |
| R | Arg | Arginine | CGT,CGC,CGA,CGG,AGA,AGG |
| S | Ser | Serine | TCT,TCC,TCA,TCG,AGT,AGC |
| T | Thr | Threonine | ACT,ACC,ACA,ACG |
| V | Val | Valine | GTT,GTC,GTA,GTG |
| W | Trp | Tryptophan | TGG |
| X | Xaa | Unknown | |
| Y | Tyr | Tyrosine | TAT, TAC |
| Z | Glu, Gln | Glutamic, | |
| | | Glutamine | GRA,GAG,CAA,CAG |
| * | End | Terminator | TAA,TAG,TGA |

Conservative substitution may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar-uncharged | C S T M |
| | | N Q |
| | Polar-charged | D E |
| | | K R |
| AROMATIC | | H F W Y |

As used herein, the term "protein" includes single-chain polypeptide molecules as well as multiple-polypeptide complexes where individual constituent polypeptides are linked by covalent or non-covalent means. As used herein, the terms "polypeptide" and "peptide" refer to a polymer in which the monomers are amino acids and are joined together through peptide or disulfide bonds. The terms subunit and domain may also refer to polypeptides and peptides having biological function. A peptide useful in the invention will at least have a target or signalling modulation capability. "Fragments" are also variants and the term typically refers to a selected region of the protein that is of interest in a binding assay and for which a binding partner is known or determinable. "Fragment" thus refers to an amino acid sequence that is a portion of a full-length polypeptide, between about 8 and about 745 amino acids in length, preferably about 8 to about 300, more preferably about 8 to about 200 amino acids, and even more preferably about 10 to about 50 or 100 amino acids in length. "Peptide" refers to a short amino acid sequence that is 10 to 40 amino acids long, preferably 10 to 35 amino acids.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

Variants of the nucleotide sequence may also be made. Such variants will preferably comprise codon optimised sequences. Codon optimisation is known in the art as a method of enhancing RNA stability and therefore gene expression. The redundancy of the genetic code means that several different codons may encode the same amino-acid. For example, Leucine, Arginine and Serine are each encoded by six different codons. Different organisms show preferences in their use of the different codons. Viruses such as HIV, for instance, use a large number of rare codons. By changing a nucleotide sequence such that rare codons are replaced by the corresponding commonly used mammalian codons, increased expression of the sequences in mammalian target cells can be achieved. Codon usage tables are known in the art for mammalian cells, as well as for a variety of other organisms.

In one embodiment of the present invention, at least one of the nucleotide sequences encoding either the target protein or the protein for T cell signalling is codon optimised for expression in mammalian cells.

In a preferred embodiment, the sequences are optimised in their entirety.

The ability of a naturally occurring or synthetic sequence to translocate the membrane or affect T cell signalling may be tested by routine methods known in the art.

Some variants of the known target proteins which retain the ability to bind to APCs have been reported in the art and these are included in the scope of the present invention, together with any which become available.

Some variants of the known proteins for T cell signalling modulation which retain this ability have been reported in the art and these are included in the scope of the present invention, together with any which become available.

Preferably, any non-native protein is prepared by use of recombinant techniques.

In a further aspect of the present invention there is provided a polynucleotide sequence encoding the fusion protein of the present invention.

"Polynucleotide" refers to a polymeric form of nucleotides of at least 10 bases in length and up to 1,000 bases or even more, either ribonucleotides or deoxyribonucleotides or a modified form of either type of nucleotide. The term includes single and double stranded forms of DNA.

These may be constructed using standard recombinant DNA methodologies. The nucleic acid may be RNA or DNA and is preferably DNA Where it is RNA, manipulations may be performed via cDNA intermediates. Generally, a nucleic acid sequence encoding the first region will be prepared and suitable restriction sites provided at the 5' and/or 3' ends. Conveniently the sequence is manipulated in a standard laboratory vector, such as a plasmid vector based on pBR322 or pUC19 (see below). Reference may be made to Molecular Cloning by Sambrook *et al.* (Cold Spring Harbor, 1989) or similar standard reference books for exact details of the appropriate techniques.

Nucleic acid encoding the second region may likewise be provided in a similar vector system.

Sources of nucleic acid may be ascertained by reference to published literature or databanks such as GenBank. Nucleic acid encoding the desired first or second sequences may be obtained from academic or commercial sources where such sources are willing to provide the material or by synthesising or cloning the appropriate sequence where only the sequence data are available. Generally this may be done by reference to literature sources which describe the cloning of the gene in question.

Alternatively, where limited sequence data are available or where it is desired to express a nucleic acid homologous or otherwise related to a known nucleic acid, exemplary nucleic acids can be characterised as those nucleotide sequences which hybridise to the nucleic acid sequences known in the art.

It will be understood by a skilled person that numerous different nucleotide sequences can encode the same target protein or protein for T cell signalling modulation used in the present invention as a result of the degeneracy of the genetic code. In addition, it is to be understood that skilled persons may, using routine techniques, make nucleotide substitutions that do not affect the target protein or protein for T cell signalling modulation encoded by the nucleotide sequence of the present invention to reflect the codon usage of any particular host organism in which the target protein or protein for Notch signalling modulation of the present invention is to be expressed.

In general, the terms "variant", "homologue" or "derivative" in relation to the nucleotide sequence used in the present invention includes any substitution of, variation of, modification of, replacement of, deletion of or addition of one (or more) nucleic acid from or to the sequence providing the resultant nucleotide sequence codes for a target protein or protein for T cell signalling modulation.

As indicated above, with respect to sequence homology, preferably there is at least 75%, more preferably at least 85%, more preferably at least 90% homology to the reference sequences. More preferably there is at least 95%, more preferably at least 98%, homology. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

The present invention also encompasses nucleotide sequences that are capable of hybridising selectively to the reference sequences, or any variant, fragment or derivative thereof or to the complement of any of the above. Nucleotide sequences are preferably at least 15 nucleotides in length, more preferably at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction (PCR) technologies.

Nucleotide sequences useful in the invention capable of selectively hybridising to the nucleotide sequences presented herein, or to their complement, will be generally at least 75%, preferably at least 85 or 90% and more preferably at least 95% or 98% homologous to the corresponding nucleotide sequences presented herein over a region of at least 20, preferably at least 25 or 30, for instance at least 40, 60 or 100 or more contiguous nucleotides. Preferred nucleotide sequences of the invention will comprise regions homologous to the nucleotide sequence, preferably at least 80 or 90% and more preferably at least 95% homologous to the nucleotide sequence.

The term "selectively hybridizable" means that the nucleotide sequence used as a probe is used under conditions where a target nucleotide sequence of the invention is found to hybridize to the probe at a level significantly above background. The background hybridization may occur because of other nucleotide sequences present, for example, in the cDNA or genomic DNA library being screened. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radiolabelling the probe, e.g. with ³²P.

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical nucleotide sequences while an intermediate (or low) stringency hybridization can be used to identify or detect similar or related polynucleotide sequences.

In a preferred aspect, the present invention covers nucleotide sequences that can hybridise to the nucleotide sequence of the present invention under stringent conditions (e.g. 65°C and O.1xSSC {1xSSC = 0.15 M NaCl, 0.015 M Na₃ Citrate pH 7.0). Where the nucleotide sequence of the invention is double-stranded, both strands of the duplex, either individually or in combination, are encompassed by the present invention. Where the nucleotide sequence is single-stranded, it is to be understood that the complementary sequence of that nucleotide sequence is also included within the scope of the present invention

Nucleotide sequences which are not 100% homologous to the sequences of the present invention but fall within the scope of the invention can be obtained in a number of ways. Other variants of the sequences described herein may be obtained for example by probing DNA libraries made from a range of sources. In addition, other vital/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of the reference nucleotide sequence under conditions of medium to high stringency. Similar considerations apply to obtaining species homologues and allelic variants of the amino acid and/or nucleotide sequences useful in the present invention.

Variants and strain/species homologues may also be obtained using degenerate PCR. which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art For example the GCG Wisconsin PileUp program is widely used. The primers used in degenerate PCR will contain one or more degenerate positions and will be used at stringency conditions lower than those used for cloning sequences with single sequence primers against known sequences.

Alternatively, such nucleotide sequences may be obtained by site directed mutagenesis of characterised sequences. This may be useful where for example silent codon changes are required to sequences to optimise codon preferences for a particular host cell in which the nucleotide sequences are being expressed. Other sequence changes may be desired in order to introduce restriction enzyme recognition sites, or to alter the activity of the target protein or protein for T cell signalling modulation encoded by the nucleotide sequences.

The nucleotide sequences such as a DNA polynucleotides useful in the invention may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer nucleotide sequences will generally be produced using recombinant means, for example using a PCR (polymerase chain reaction) cloning techniques. This will involve making a pair of primers (e.g. of about 15 to 30 nucleotides) flanking a region of the targeting sequence which it is desired to clone, bringing the primers into contact with mRNA or cDNA obtained from an animal or human cell, performing a polymerase chain reaction (PCR) under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

According to further aspects of the present invention there is provided an expression vector comprising the polynucleotide sequence of the present invention; a host cell transformed with the expression vector of the present invention; a method for preparing a fusion protein of the present invention comprising culturing the host cell of the present invention under conditions which provide for the expression of the fusion protein; a method of targeting a protein for T cell signalling modulation to an APC comprising exposing an APC to a conjugate according to the present invention; a conjugate prepared by the method of the present invention; a pharmaceutical composition comprising the conjugate of the present invention, particularly for use in the treatment of T-cell mediated disease; and use of the conjugate of the present invention in the preparation of a medicament for the prevention and/or treatment of disease or infection, particularly a T-cell mediated disease.

The conjugates of the present invention may be prepared by any methods known in the art.

The present invention also relates to vectors which comprise a polynucleotide useful in the present invention, host cells which are genetically engineered with vectors of the invention and the production of polypeptides useful in the present invention by such techniques.

For recombinant production, host cells can be genetically engineered to incorporate expression systems or polynucleotides of the invention. Introduction of a polynucleotide into the host cell can be effected by methods described in many standard laboratory manuals, such as Davis *et al* and Sambrook *et al,* such as calcium phosphate transfection, DEAE-dextran mediated transfection, transvection, microinjection, cationic lipid-mediated transfection, electroporation, transduction, scrape loading, ballistic introduction and infection. In will be appreciated that such methods can be employed *in vitro* or *in vivo* as drug delivery systems.

Representative examples of appropriate hosts include bacterial cells, such as streptococci, staphylococci, *E. coli,* streptomyces and *Bacillus subtilis* cells; fungal cells, such as yeast cells and *Aspergillus* cells; insect cells such as *Drosophila* S2 and *Spodoptera* Sf9 cells; animal cells such as CHO, COS, NSO, HeLa, C127, 3T3, BHK, 293 and Bowes melanoma cells; and plant cells.

A great variety of expression systems can be used to produce a polypeptide useful in the present invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression, in this regard. The appropriate DNA sequence may be inserted into the expression system by any of a variety of well-known and routine techniques, such as, for example, those set forth in Sambrook *et al.*

For secretion of the translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Conjugates of the invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding protein may be employed to regenerate active conformation when the polypeptide is denatured during isolation and/or purification.

Chemically coupled sequences can be prepared from individual proteins sequences and coupled using known chemically coupling techniques. The conjugate can be assembled using conventional solution- or solid-phase peptide synthesis methods, affording a fully protected precursor with only the terminal amino group in deprotected reactive form. This function can then be reacted directly with a protein for T cell signalling modulation or a suitable reactive derivative thereof. Alternatively, this amino group may be converted into a different functional group suitable for reaction with a cargo moiety or a linker. Thus, e.g. reaction of the amino group with succinic anhydride will provide a selectively addressable carboxyl group, while further peptide chain extension with a cysteine derivative will result in a selectively addressable thiol group. Once a suitable selectively addressable functional group has been obtained in the delivery vector precursor, a protein for T cell signalling modulation or a derivative thereof may be attached through e.g. amide, ester, or disulphide bond formation. Cross-linking reagents which can be utilized are discussed, for example, in Neans, G.E. and Feeney, R.E., Chemical Modification of Proteins, Holden-Day, 1974, pp. 39-43.

As discussed above the target protein and protein for T cell signalling modulation may be linked directly or indirectly via a cleavable linker moiety. Direct linkage may occur through any convenient functional group on the protein for T cell signalling modulation such as a hydroxy, carboxy or amino group. Indirect linkage which is preferable, will occur through a linking moiety. Suitable linking moieties include bi- and multi-functional alkyl, aryl, aralkyl or peptidic moieties, alkyl, aryl or aralkyl aldehydes acids esters and anyhdrides, sulphydryl or carboxyl groups, such as maleimido benzoic acid derivatives, maleimido proprionic acid derivatives and succinimido derivatives or may be derived from cyanuric bromide or chloride, carbonyldiimidazole, succinimidyl esters or sulphonic halides and the like. The functional groups on the linker moiety used to form covalent bonds between linker and protein for T cell signalling modulation on the one hand, as well as linker and target protein on the other hand, may be two or more of, e.g., amino, hydrazino, hydroxyl, thiol, maleimido, carbonyl, and carboxyl groups, etc. The linker moiety may include a short sequence of from 1 to 4 amino acid residues that optionally includes a cysteine residue through which the linker moiety bonds to the target protein.

In accordance with the present invention each target protein may be linked to at least one protein for T cell signalling modulation. In a further embodiment, the target protein is prepared such as to facilitate linkage to more than one protein for T cell signalling modulation, each protein for T cell signalling modulation being the same or different. For example, the target protein may comprise components that themselves facilitate the attachment of more than one protein for T cell signalling modulation such as derivatives of naturally occurring amino acids or insertion of a multi-valent synthetic amino acid, or it may be specifically adapted to do so for example by a network of branched lysine residues that may be attached to the target protein as a linking group and each lysine residue may then be attached to a protein for T cell signalling modulation. In this manner a single target protein may carry up to 32 proteins far T cell signalling modulation, preferably from 2 to 10 or more preferably from 4 to 5 proteins for T cell signalling modulation. In this further embodiment each protein for T cell signalling modulation may be directly or indirectly linked to the carrier moiety. When more than one different type of protein for T cell signalling modulaiton is attached, it is possible to co-ordinabe the ratios and dosages of the individual drugs to facilitate the administration of specific protein combinations.

Stable aggregates, having particle sizes for example in the range of from 0.1 to 5 microns, may be formed by mixing the protein for T cell signalling modulation with the target protein. Ratios of from 2:1 to 1:1 of target protein to protein for T cell signalling modulation are preferred.

In a further embodiment, the conjugate may further comprise a targeting moiety. The targeting moiety is capable of directing the target protein to the specific cell type to which it is preferable for the protein for T cell signalling modulation to function. Thus, the targeting moiety acts as an address system biasing the body's natural distribution of drugs or the conjugate to a particular cell type. The targeting moiety may be attached to the protein for T cell signalling modulation or more preferably to the target protein and will direct the conjugate to a desired site, upon arrival at which the target protein will facilitate the cellular internalisation of the protein for T cell signalling modualtion. Suitable targeting moieties include, for example, cell specific antibodies or antibody fragments such as phage-displayed ScFv and other peptide sequences identified by E Ruoslahti et al. in US Patent 5,622,699; Pasqualini, R, Ruoslahti E; Ruoslahti E; and Arap, W, Pasqualini, R, Ruoslahti, B.

A stabilizing agent, which serves to increase conjugate stability and uptake, can optionally be brought into contact with cells, in conjunction with the conjugate. For example, metal ions which bind to tat protein and increase its stability and uptake, can be used for this purpose.

In a further embodiment of this invention, a lysosomotrophic agent is provided extracellularly in conjunction with the conjugate, in order to enhance uptake by cells. The lysosomotrophic agent can be used alone or in conjunction with a stabilizer. For example lysosomotrophic agents such as chloroquine, monensin, amantadine and methylamine, which have been shown to increase uptake of tat in some cells by a few hundred fold, can be used for this purpose.

In another embodiment, a basic peptide, such as tat 38-58 or protamine, is provided extracellularly with the conjugate to enhance its uptake. Such basic peptides can also be used alone, in combination or with stabilizing agents or lysosomotrophic agents.

The conjugates of the present invention can also be used to raise antibodies which can be used in diagnostic and monitoring specific binding assays using conventional techniques, for example, monitoring the localisation of the conjugates themselves or their components.

In accordance with yet another embodiment of the present invention, there are provided antibodies specifically recognising and binding the conjugates according to the invention. More preferably, however, the antibodies are specific for the second sequence of the conjugates. Advantageously, the second sequence of the conjugate is recognised by the antibodies when in its natural context. Thus, where the second sequence is an isolated fragment or domain from a protein for T cell signalling modulation, that fragment or domain is recognised by the antibodies of the invention in the context of the whole of the larger protein.

The invention moreover provides a method for preparing an immunoglobulin, comprising the steps of:
a) immunising an animal with a conjugate according to the present invention; and
b) recovering immunoglobulin specific for a region of the conjugate from the serum of the animal.

The antibodies (or immunoglobulins) may be isolated in the form of a crude preparation, i.e. an antiserum, by affinity chromatography against the conjugate. Alternatively, monoclonal antibodies may be prepared according to standard techniques in the art and purified.

The therapeutic effect resulting from the administration of the conjugate may arise from the intact conjugate or any of the dissociated proteins for T cell signalling modulation alone or bound to the linker, part of the linker or the linker and part of the target protein.

In the preferred embodiment the therapeutic effect results from a protein for Notch signalling. A detailed description of the Notch signalling pathway and conditions affected by it may be found in our WO98/20142, WO00/36089 and PCT/GB00/04391.

Diseased or infectious states that may be described as being mediated by T cells include, but are not limited to, any one or more of asthma, allergy, graft rejection, autoimmunity, tumour induced aberrations to the T cell system and infectious diseases such as those caused by Plasmodium species, Microfilariae, Helminths, Mycobacteria, HIV, Cytomegalovirus, Pseudomonas, Toxoplasma, Echinococcus, Haemophilus influenza type B, measles, Hepatitis C or Toxicara. Thus particular conditions that may be treated or prevented which are mediated by T cells include multiple schlerosis, rheumatoid arthritis and diabetes. The present invention may also be used in organ transplantation or bone marrow transplantation.

As indicated above, the present invention is useful in treating immune disorders such as autoimmune diseases or graft rejection such as allograft rejection.

Examples of disorders that may be treated include a group commonly called autoimmune diseases. The spectrum of autoimmune disorders ranges from organ specific diseases (such as thyroiditis, insulitis, multiple sclerosis, iridocyclitis, uveitis, orchitis, hepatitis, Addison's disease, myasthenia gravis) to systemic illnesses such as rheumatoid arthritis or lupus erythematosus. Other disorders include immune hyperreactivity, such as allergic reactions.

In more detail: Organ-specific autoimmune diseases include multiple sclerosis, insulin dependent diabetes mellitus, several forms of anemia (aplastic, hemolytic), autoimmune hepatitis, thyroiditis, insulitis, iridocyclitis, skleritis, uveitis, orchitis, myasthenia gravis, idiopathic thrombocytopenic purpura, inflammatory bowel diseases (Crohn's disease, ulcerative colitis).

Systemic autoimmune diseases include: rheumatoid arthritis, juvenile arthritis, scleroderma and systemic sclerosis, sjogren's syndrom, undifferentiated connective tissue syndrome, antiphospholipid syndrome, different forms of vasculitis (polyarteritis nodosa, allergic granulomatosis and angütis, Wegner's granulomatosis, Kawasaki disease, hypersensitivity vasculitis, Henoch-Schoenlein purpura, Behcet's Syndrome, Takayasu arteritis, Giant cell arteritis, Thrombangütis obliterans), lupus erythematosus, polymyalgia rheumatica, essentiell (mixed) cryoglobulinemia, Psoriasis vulgaris and psoriatic arthritis, diffus fasciitis with or without eosinophilia, polymyositis and other idiopathic inflammatory myopathies, relapsing panniculitis, relapsing polychondritis, lymphomatoid granulomatosis, erythema nodosum, ankylosing spondylitis, Reiter's syndrome, different forms of inflammatory dermatitis.

A more extensive list of disorders includes: unwanted immune reactions and inflammation including arthritis, including rheumatoid arthritis, inflammation associated with hypersensitivity, allergic reactions, asthma, systemic lupus erythematosus, collagen diseases and other autoimmune diseases, inflammation associated with atherosclerosis, arteriosclerosis, atherosclerotic heart disease, reperfusion injury, cardiac arrest, myocardial infarction, vascular inflammatory disorders, respiratory distress syndrome or other cardiopulmonary diseases, inflammation associated with peptic ulcer, ulcerative colitis and other diseases of the gastrointestinal tract, hepatic fibrosis, liver cirrhosis or other hepatic diseases, thyroiditis or other glandular diseases, glomerulonephritis or other renal and urologic diseases, otitis or other oto-rhino-laryngological diseases, dermatitis or other dermal diseases, periodontal diseases or other dental diseases, orchitis or epididimo-orchitis, infertility, orchidal tramma or other immune-related testicular diseases, placental dysfunction, placental insufficiency, habitual abortion, eclampsia, pre-eclampsia and other immune and/or inflammatory-related gynaecological diseases, posterior uveitis, intermediate uveitis, anterior uveitis, conjunctivitis, chorioretinitis, uveoretinitis, optic neuritis, intraocular inflammation, e.g. retinitis or cystoid macular oedema, sympathetic ophthalmia, scleritis, retinitis pigmentosa, immune and inflammatory components of degenerative fondus disease, inflammatory components of ocular trauma, ocular inflammation caused by infection, proliferative vitreo-retinopathies, acute ischaemic optic neuropathy, excessive scarring, e.g. following glaucoma filtration operation, immune and/or inflammation reaction against ocular implants and other immune and inflammatory-related ophthalmic diseases, inflammation associated with autoimmune diseases or conditions or disorders where, both in the central nervous system (CNS) or in any other organ, immune and/or inflammation suppression would be beneficial, Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, inflammatory components of stokes, post-polio syndrome, immune and inflammatory components of psychiatric disorders, myelitis, encephalitis, subacute sclerosing pan-encephalitis, encephalomyelitis, acute neuropathy, subacute neuropathy, chronic neuropathy, Guillaim-Barre syndrome, Sydenham chora, myasthenia gravis, pseudo-tumour cerebri, Down's Syndrome, Huntington's disease, amyotrophic lateral sclerosis, inflammatory components of CNS compression or CNS trauma or infections of the CNS, inflammatory components of muscular atrophies and dystrophies, and immune and inflammatory related diseases, conditions or disorders of the central and peripheral nervous systems, post-traumatic inflammation, septic shock, infectious diseases, inflammatory complications or side effects of surgery or organ, inflammatory and/or immune complications and side effects of gene therapy, e.g. due to infection with a viral carrier, or inflammation associated with AIDS, to suppress or inhibit a humoral and/or cellular immune response, to treat or ameliorate monocyte or leukocyte proliferative diseases, e.g. leukaemia, by reducing the amount of monocytes or lymphocytes, for the prevention and/or treatment of graft rejection in cases of transplantation of natural or artificial cells, tissue and organs such as cornea, bone marrow, organs, lenses, pacemakers, natural or artificial skin tissue.

The present invention is also useful in cancer therapy. The present invention is especially useful in relation to adenocarcinomas such as: small cell long cancer, and cancer of the kidney, uterus, prostrate, bladder, ovary, colon and breast.

The present invention is also useful in methods for altering the fate of a cell, tissue or organ type by altering Notch pathway function in the cell. Thus, the present application has application in the treatment of malignant and pre-neoplastic disorders.

The present invention is especially useful in relation to adenocarcinomas such as: small cell hmg cancer, and cancer of the kidney, uterus, prostrate, bladder, ovary, colon and breast. For example, malignancies which may be treatable according to the present invention include acute and chronic leukemias, lymphomas, myelomas, sarcomas such as Fibrosarcoma, myxosarcoma, liposarcoma, lymphangioendotheliosarcoma, angiosarcoma, endotheliosarcoma, chondrosarcoma, osteogenic sarcoma, chordoma, lymphangiosarcoma, synovioma, mesothelioma, leimyosarcoma, rhabdomyosarcoma, colon carcinoma, ovarian cancer, prostate cancer, pancreatic cancer, breasy cancer, squamous cell carcinoma, basal cell carcinoma, adenocarcinoma, sewat gland carcinoma, sebaceous gland carcinoma, papillary carcinoma, papillary adenocarcinomas, cystadenocarcinoma, medullary carcinoma, bronchogenic carcinoma, choriocarcinoma, renal cell carcinoma, hepatoma, bile duct carcinoma serninoma, embryonal carcinoma, cervical cancer, testicular tumour, lung carcinoma, small cell lung carcinoma, bladder carcinoma, epithelial carcinoma, glioma, astrocytoma, ependymoma, pinealoma, hemangioblastoma, acoustic neuoma, medulloblastoma, craniopharyngioma, oligodendroglioma, menangioma, melanoma, neutroblastoma and retinoblastoma.

The present invention may also have application in the treatment of nervous system disorders. Nervous system disorders which may be treated according to the present invention include neurological lesions including traumatic lesions resulting from physical injuries; ischaemic lesions; malignant lesions; infectious lesions such as those caused by HIV, herpes zoster or herpes simplex virus, Lyme disease, tuberculosis or syphilis; degenerative lesions and diseases and demyelinated lesions.

The present invention may be used to treat, for example, diabetes (including diabetic neuropathy, Bell's palsy), systemic lupus erythematosus, sarcoidosis, multiple sclerosis, human immunodeficiency virus-associated myelopathy, transverse myelopathy or various etiologies, progressive multifocal leukoencephalopathy, central pontine myelinolysis, Parkinson's disease, Alzheimer's disease, Huntington's chorea, amyotrophic lateral sclerosis, cerebral infarction or ischemia, spinal cord infarction or ischemia, progressive spinal muscular atrophy, progressive bulbar palsy, primary lateral sclerosis, infantile and juvenile muscular atrophy, progressive bulbar paralysis of childhood (Fazio-Londe syndrome), poliomyelitis and the post polio syndrome, and Hereditary Motorsensory Neuropathy (Charcot-Marie-Tooth Disease).

The present invention may further be useful in the promotion of tissue regeneration and repair. The present invention, therefore, may also be used to treat diseases associated with defective tissue repair and regeneration such as, for example, cirrhosis of the liver, hypertrophic scar formation and psoriasis. The invention may also be useful in the treatment of neutropenia or anemia and in techniques of organ regeneration and tissue engineering.

We have now found that the use of the conjugate of the present invention may prevent and/or promote regression of the above-mentioned diseases.

The present invention can be used to deliver a protein for T cell signal modulation into cells, particularly the cell nucleus, *in vitro* or *in vivo.* In *in vitro* applications, the conjugate may be added to a culture medium of the target cells. The conjugate can also be combined with a cell sample obtained from an individual in order to introduce the protein for T cell signalling modulation into cells present in the sample. After treatment in this manner the sample can be returned to the individual. The conjugate can also be administered *in vivo.* For example cells that synthesise the conjugate can be produced and implanted into an individual. In a further embodiment, the conjugate can be used much like a conventional therapeutic agent and can be a component of a pharmaceutical composition.

For example, delivery can be carried out *in vitro* by adding a conjugate to cultured cells, by producing cells that synthesize conjugate or by combining a sample (e.g., blood, bone marrow) obtained from an individual with the conjugate, under appropriate conditions. Thus, the target cells may be *in vitro* cells, Le., cultured animals cells, human cells or micro-orzanisms. Delivery can be carried out *in vivo* by administering the conjugate to an individual in whom it is to be used for diagnostic, preventative or therapeutic purposes. The target cells may be *in vivo* cells, i.e., cells composing the organs or tissues of living animals or humans, or microorganisms found in living animals or humans.

The conjugate may be administered by viral or non-viral techniques. Viral delivery mechanisms include but are not limited to adenoviral vectors, adeno-associated viral (AAV) vectos, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof. The routes for such delivery mechanisms include but are not limited to mucosal, nasal, oral, parenteral, gastrointestinal, topical, or sublingual routes. The conjugates of the present invention may be adminstered by conventional DNA delivery techniques, such as DNA vaccination etc., or injected or otherwise delivered with needleless systems, such as ballistic delivery on particles, such as gold, coated with the DNA for delivery to the epidermis or other sites such as mucosal surfaces.

The conjugates of the present invention are typically formulated for administration to patients with a pharmaceutically acceptable carrier or diluent to produce a pharmaceutical composition. The formulation will depend upon the nature of the compound identified and the route of administration but typically they can be formulated for topical, parenteral, intramuscular, intravenous, intra-peritoneal, intranasal inhalation, lung inhalation, intradermal or intra-articular administration. The conjugate may be used in an injectable form. It may therefore be mixed with any vehicle which is pharmaceutically acceptable for an injectable formulation, preferably for a direct injection at the site to be treated, although it may be administered systemically.

The pharmaceutically acceptable carrier or diluent may be, for example, sterile isotonic saline solutions, or other isotonic solutions such as phosphate-buffered saline. The conjugates of the present invention may be admixed with any suitable binder(s), lubricant(s), suspending agent(s), coating agent(s), solubilising agent(s). It is also preferred to formulate the compound in an orally active form.

In general, a therapeutically effective daily oral or intravenous dose of the conjugate of the invention is likely to range from 0.01 to 50 mg/kg body weight of the subject to be treated, preferably 0.1 to 20 mg/kg. The conjugate may also be administered by intravenous infusion, at a dose which is likely to range from 0.001-10 mg/kg/hr.

Tablets or capsules of the conjugates may be administered singly or two or more at a time, as appropriate. It is also possible to administer the conjugates in sustained release formulations.

Typically, the physician will determine the actual dosage which will be most suitable for an individual patient and it will vary with the age, weight and response of the particular patient. The above dosages are exemplary of the average case. There can, of course, be individual instances where higher or lower dosage ranges are merited, and such are within the scope of this invention.

Alternatively, the conjugates of the invention can be administered by inhalation or in the form of a suppository or pessary, or they may be applied topically in the form of a lotion, solution, cream, ointment or dusting powder. An alternative means of transdermal administration is by use of a skin patch. For example, they can be incorporated into a cream consisting of an aqueous emulsion of polyethylene glycols or liquid paraffin. They can also be incorporated, at a concentration of between 1 and 10% by weight, into an ointment consisting of a white wax or white soft paraffin base together with such stabilisers and preservatives as may be required.

For some applications, preferably the conjugates are administered orally in the form of tablets containing excipients such as starch or lactose, or in capsules or ovules either alone or in admixture with excipients, or in the form of elixirs, solutions or suspensions containing flavouring or colouring agents.

The conjugates can also be injected parenterally, for example intracavernosally, intravenously, intramuscularly or subcutaneously. In this case, the conjugates will comprise a suitable carrier or diluent.

For parenteral administration, the conjugates are best used in the form of a sterile aqueous solution which may contain other substances, for example enough salts or monosaccharides to make the solution isotonic with blood.

For buccal or sublingual administration the conjugates may be administered in the form of tablets or lozenges which can be formulated in a conventional manner.

For oral, parenteral, buccal and sublingual administration to subjects (such as patients), the daily dosage level of the conjugates of the present invention and their pharmaceutically acceptable salts and solvates may typically be from 10 to 500 mg (in single or divided doses). Thus, and by way of example, tablets or capsules may contain from 5 to 100 mg of active compound for administration singly, or two or more at a time, as appropriate. As indicated above, the physician will determine the actual dosage which will be most suitable for an individual patient and it will vary with the age, weight and response of the particular patient. It is to be noted that whilst the above-mentioned dosages are exemplary of the average case there can, of course, be individual instances where higher or lower dosage ranges are merited and such dose ranges are within the scope of this invention.

The routes of administration and dosages described are intended only as a guide since a skilled practitioner will be able to determine readily the optimum route of administration and dosage for any particular patient depending on, for example, the age, weight and condition of the patient.

The term treatment or therapy as used herein should be taken to encompass diagnostic and prophylatic applications.

The treatment of the present invention includes both human and veterinary applications.

The conjugates of the present invention provide several advantages over known delivery systems. These advantages include improved efficacy compared to conventional treatments, improved cellular uptake of the therapeutic agent, improved water solubility, reduction of side effects and cellular bioavailability and decreased occurrence of drug resistance.

Various preferred features and embodiments of the present invention will now be described in more detail by way of non-limiting example and with reference to the accompanying drawings, in which:

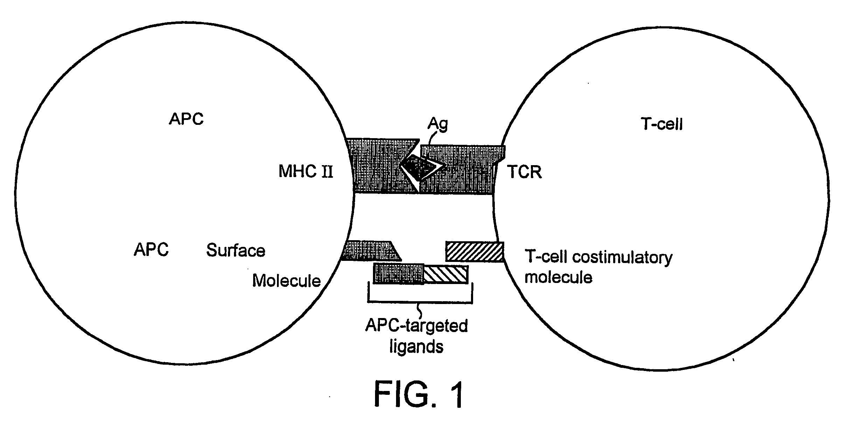

Figure 1 shows a schematic representation of an embodiment of the present invention in which the first sequence is targeted to an APC surface moelcule and the second sequence is a modulator of a T cell costimulatory molecule;
Figure 2 shows a schematic representation of Notch;
Figure 3 shows a schematic representation of NotchIC;
Figures 4 and 5 show schematic representations of the Notch signalling pathway;
Figure 6 is a schematic representation of superantigen recognition of MHC and TCR;

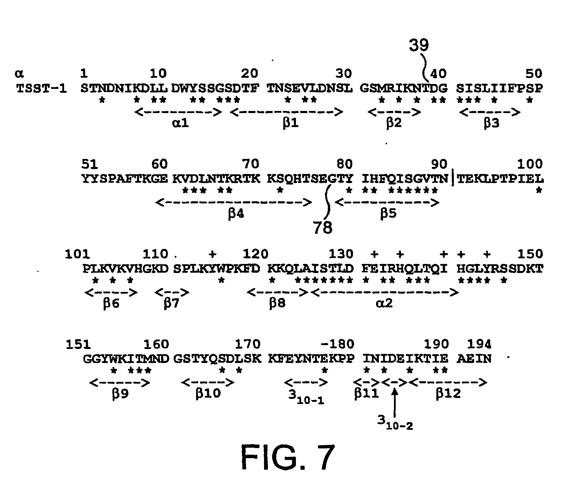

Figure 7 shows the amino acid sequence of TSST-1;

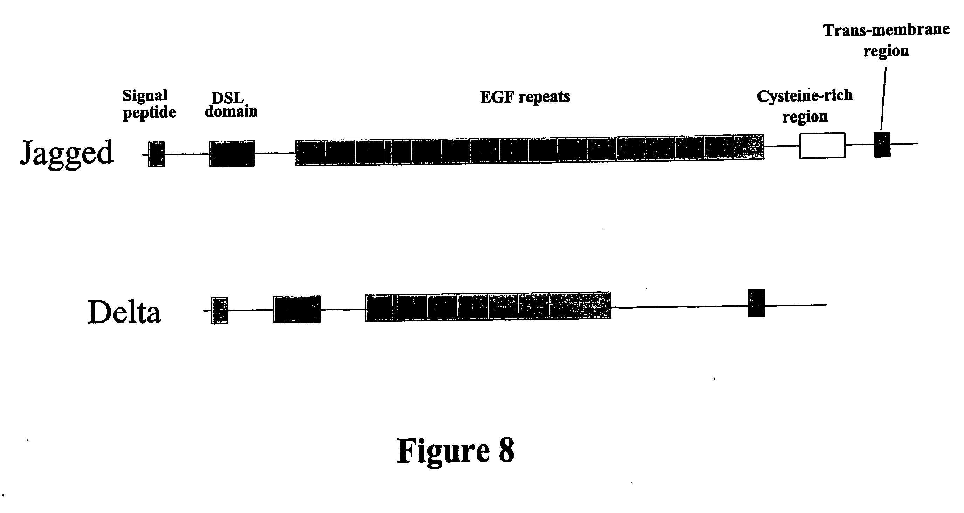

Figure 8 shows schematic representations of the Notch ligands Jagged and Delta;
Figure 9 shows aligned amino acid sequences of DSL domains from various Drosophila and mammalian Notch ligands;
Figure 10 shows amino acid sequences of human Delta-1, Delta-3 and Delta-4; and
Figure 11 shows amino acid sequences of human Jagged-1 and Jagged-2.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; and, D. M. J. Lilley and J. E. Dahlberg, 1992, Methods of Enzymology: DNA Structure Part A: Synthesis and Physical Analysis of DNA Methods in Enzymology, Academic Press. Each of these general texts is herein incorporated by reference.

### POLYPEPTIDE WHICH IS CAPABLE OF BINDING TO A MHC CLASS II MOLECULE

In a preferred embodiment the conjugates of the present invention are characterised by a structure that is recognised by MHC class II molecules as being a superantigen.

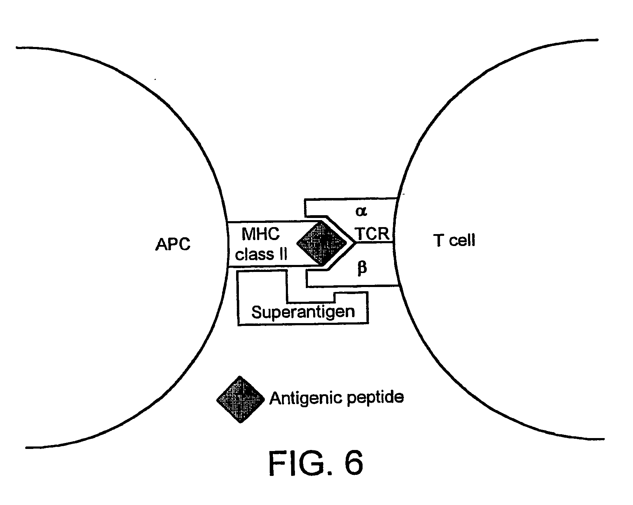

In more detail, while a single peptide antigen may be recognised by or is immunogenic for a small number of T cell clones, a special category of antigens, known as superantigens, have capacity to stimulate multiple T cell clones. Superantigens, which have been identified thus far as bacterial and viral glycoproteins, are superstimulators of T cells because they are capable of binding to a large number of T cell receptor Vβ sequences, as well as to MHC class II molecules outside of the peptide presentation groove. As shown in Fig 6, the binding of superantigens to relatively nonpolymorphic regions of MHC and TCR molecules promotes adherence of T cells to antigen-presenting cells, irrespective of antigen specificity of the TCR. Such cross-linking of TCR with MHC molecules leads to activation of multiple clones of CD4+ cells.

Superantigens are a unique class of antigen from bacteria and viruses that have the ability to bind to the TCR-MHC complex in a less stringent fashion and to activate a large number of T cells, resulting in various severe illnesses such as food poisoning and toxic shock syndrome.

Although most antigenic peptides are presented to the TCR by inserting between two helices of the MHC molecules, the superantigens bind to the lateral surfaces of MHC II. Further more, they bind directly to the portion of TCR encoded by the Vβ genes that are not part of the antigen binding sites (CDR3). It should be noted that binding of superantigen to TCR αβ is independent of the α chain and the DJ segments of the Vβ chains, the segment that encodes CDR3.

Thus, superantigens are presented to T cells in an MHC-unrestricted manner and are only presented to T cells expressing a T cell receptor possessing a specific variable β gene product. Thus, many T cells are activated in an antigen-nonspecific and MHC-unrestricted manner.

Preferred superantigens are selected from the group of staphylococcal enterotoxins (SEs), such as SEA, SEB, SEC, SED, SEE and SEH, toxoids, and active fragments thereof. The superantigens may include other microbial products, such as bacterial as well as viral, such as products from staphylococcal strains, e.g. Toxic Shock syndrome toxins (TSST-1), and from streptococcal strains, e.g. the pyrogenic exotoxins (SPE), such as SPEA, SPEC and SSA.

In a particularly preferred embodiment of the present invention use is made of TSST-1. A review of the binding domains of TSST-1 is provided in Wahlstein and Ramakrishnan (1998) which confirms that structurally TSST-1 is composed of two distinct domains and localising the MHC II contact residues to within the TSST-1 N-terminal β-barrel and corresponds to N-terminal residues 1-87. The amino acid sequence of TSST-1 is shown in Fig 6. Kum 2000 reports TSST-1 residues G31/S32 to be important for MHC class II binding, but that other binding regions exist such as a discontinuous epitope comprising of regions within both the beta 1/beta 2 and beta 3/beta 4 loops. Rubinchik and Chow (2000) discloses that TSST-1 residues 47-64 is useful as a MHC class II binding domain. In one embodiment the sequence used comprises approx the first 90 amino acids or TSST-1 or a sequence having at least 50%, preferably at least 70%, preferably at least 90%, perfaerably at least 95% amino acid sequence similarity, preferably identity to such a sequence.

A review of the SpeA MHC class II binding domain is provided by Papageorgiou et al. (1999). Roussel et al (1997) reports that in SpeC MHC class II binding occurs through a zinc binding site that is analogous to the site in SpeA.

Sundstrom et al (1996) reports that SED is dependent upon a Zn2+ homodimer for high affinity interactions with MHC class II molecules.

Use may also be made of MHC class II binding domains from viral proteins such as herpesvirus saimiri (HVS). An example of an HVS protein capable of binding a MHC class II molecule is discloses in US Patent 5.716,623. US Patent No. 5,925,734 discloses viral proteins from Epstein Barr virus (EBV) which bind to MHC class II molecules.

It will be appreciated that one can apply structural-based drug design to develop synthetic superantigens with improved MHC class II binding ability.

It will be appreciated that any of the above MHC class II binding domains, or any which become available, may be utilised in the present invention,

### PROTEIN FOR NOTCH SIGNALLING MODULATION

### a. Polypeptides and Polynucleotides for Notch signalling Transduction:

By a protein which is for Notch signalling transduction is meant a molecule which participates in signalling through Notch receptors including activation of Notch, the downstream events of the Notch signalling pathway, transcriptional regulation of downstream target genes and other non-transcriptional downstream events (e.g. post-translational modification of existing proteins). More particularly, the protein is a domain that allows activation of target genes of the Notch signalling pathway, or a polynucleotide sequence which codes therefor.

A very important component of the Notch signalling pathway is Notch receptor/Notch ligand interaction. Thus Notch signalling may involve changes in expression, nature, amount or activity of Notch ligands or receptors or their resulting cleavage products. In addition, Notch signalling may involve changes in expression, nature, amount or activity of Notch signalling pathway membrane proteins or G-proteins or Notch signalling pathway enzymes such as proteases, kinases (e.g. serine/threonine kinases), phosphatases, ligases (e.g. ubiquitin ligases) or glycosyltraosferases. Alternatively the signalling may involve changes in expression, nature, amount or activity of DNA binding elements such as transcription factors.

In the present invention Notch signalling means specific signalling, meaning that the signal detected results substantially or at least predominantly from the Notch signalling pathway, and preferably from Notch/Notch ligand interaction, rather than any other significant interfering or competing cause, such as cytokine signalling. In one embodiment the term "Notch signalling" excludes cytokine signalling. The Notch signalling pathway is described in more detail below.

Proteins or polypeptides may be in the form of the "mature" protein or may be a part of a larger protein such as a fusion protein or precursor. For example, it is often advantageous to include an additional amino acid sequence which contains secretory or leader sequences or pro-sequences (such as a HIS oligomer, immunoglobulin Fc, glutathione S-transferase, FLAG etc) to aid in purification. Likewise such an additional sequence may sometimes be desirable to provide added stability during recombinant production. In such cases the additional sequence may be cleaved (eg chemically or enzymatically) to yield the final product. In some cases, however, the additional sequence may also confer a desirable pharmacological profile (as in the case of IgFc fusion proteins) in which case it may be preferred that the additional sequence is not removed so that it is present in the final product as administered.

In one embodiment the Notch ligand which activates Notch may be expressed on a cell or cell membrane, suitably derived from a cell.

The Notch signalling pathway directs binary cell fate decisions in the embryo. Notch was first described in *Drosophila* as a transmembrane protein that functions as a receptor for two different ligands, Delta and Serrate. Vertebrates express multiple Notch receptors and ligands (discussed below). At least four Notch receptors (Notch-1, Notch 2, Notch-3 and Notch-4) have been identified to date in human cells.

Notch proteins are synthesized as single polypeptide precursors that undergo cleavage via a Furin-like convertase that yields two polypeptide chains that are further processed to form the mature receptor. The Notch receptor present in the plasma membrane comprises a heterodimer of two Notch proteolytic cleavage products, one comprising an N-terminal fragment consisting of a portion of the extracellular domain, the transmembrane domain and the intracellular domain, and the other comprising the majority of the extracellular domain. The proteolytic cleavage step of Notch to activate the receptor occurs in the Golgi apparatus and is mediated by a furin-like convertase.

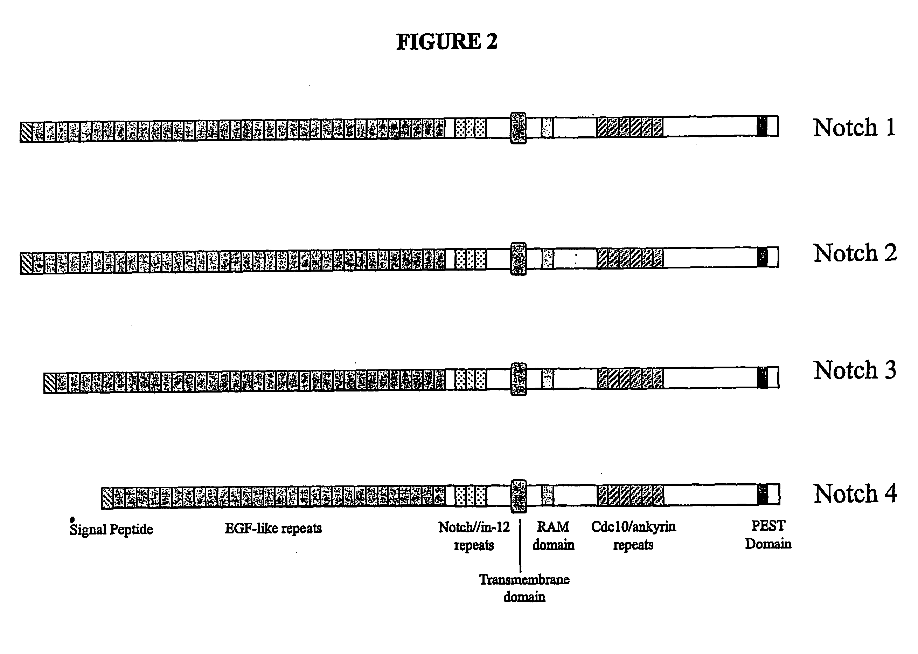

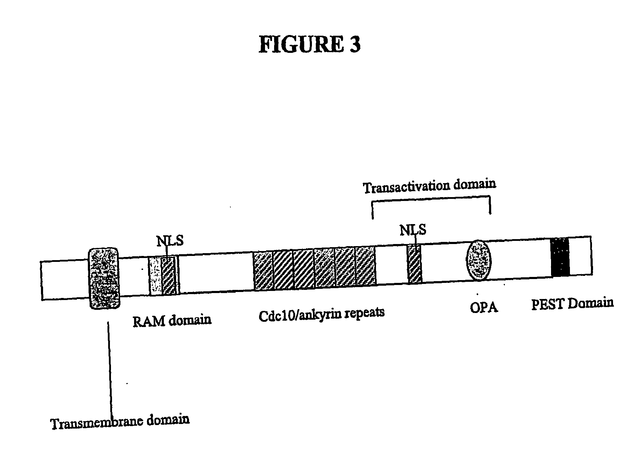

Notch receptors are inserted into the membrane as disulphide-linked heterodimeric molecules consisting of an extracellular domain containing up to 36 epidermal growth factor (EGF)-like repeats [Notch 1/2 = 36, Notch 3 = 34 and Notch 4 = 29] and a transmembrane subunit that contains the cytoplasmic domain. The cytoplasmic domain of Notch contains six ankyrin-like repeats, a polyglutamine stretch (OPA) and a PEST sequence. A further domain termed RAM23 lies proximal to the ankyrin repeats and is involved in binding to a transcription factor, known as Suppressor of Hairless [Su(H)] in *Drosophila* and CBF1 in vertebrates (Tamura). The Notch ligands also display multiple EGF-like repeats in their extracellular domains together with a cysteine-rich DSL (Delta-Serrate Lag2) domain that is characteristic of all Notch ligands (Artavanis-Tsakonas). Schematic representations of Notch and the Notch intracellular domain are shown in Figures 2 and 3.

The Notch receptor is activated by binding of extracellular ligands, such as Delta, Serrate and Scabrous, to the EGF-like repeats of Notch's extracellular domain. Delta requires cleavage for activation. It is cleaved by the ADAM disintegrin metalloprotease Kuzbanian at the cell surface, the cleavage event releasing a soluble and active form of Delta. An oncogenic variant of the human Notch-1 protein, also known as TAN-1, which has a truncated extracellular domain, is constitutively active and has been found to be involved in T-cell lymphoblastic leukemias.

The cdc10/ankyrin intracellular-domain repeats mediate physical interaction with intracellular signal transduction proteins. Most notably, the cdc10/ankyrin repeats interact with Suppressor of Hairless [Su(H)]. Su(H) is the *Drosophila* homologue of C-promoter binding factor-1 [CBF-1], a mammalian DNA binding protein involved in the Epstein-Barr virus-induced immortalization of B-cells. It has been demonstrated that, at least in cultured cells, Su(H) associates with the cdc10/ankyrin repeats in the cytoplasm and translocates into the nucleus upon the interaction of the Notch receptor with its ligand Delta on adjacent cells. Su(H) includes responsive elements found in the promoters of several genes and has been found to be a critical downstream protein in the Notch signalling pathway. The involvement of Su(H) in transcription is thought to be modulated by Hairless.

The intracellular domain of Notch (NotchIC) also has a direct nuclear function (Lieber). Recent studies have indeed shown that Notch activation requires that the six cdc10/ankyrin repeats of the Notch intracellular domain reach the nucleus and participate in transcriptional activation. The site of proteolytic cleavage on the intracellular tail of Notch has been identified between gly1743 and val1744 (termed site 3, or S3) (Schroeter). It is thought that the proteolytic cleavage step that releases the cdc10/ankyrin repeats for nuclear entry is dependent on Presenilin activity.

The intracellular domain has been shown to accumulate in the nucleus where it forms a transcriptional activator complex with the CSL family protein CBF1 (suppressor of hairless, Su(H) in *Drosophila,* Lag-2 in *C. elegans)* (Schroeter; StruhI). The NotchIC-CBF1 complexes then activate target genes, such as the bHLH proteins HES (hairy-enhancer of split like) 1 and 5 (Weinmaster). This nuclear function of Notch has also been shown for the mammalian Notch homologue (Lu).

S3 processing occurs only in response to binding of Notch ligands Delta or Serrate/Jagged. The post-translational modification of the nascent Notch receptor in the Golgi (Munro; Ju) appears, at least in part, to control which of the two types of ligand is expressed on a cell surface. The Notch receptor is modified on its extracellular domain by Fringe, a glycosyl transferase enzyme that binds to the Notch/Lin motif. Fringe modifies Notch by adding *O*-linked fucose groups to the EGF-like repeats (Moloney; Brückner). This modification by Fringe does not prevent ligand binding, but may influence ligand induced conformational changes in Notch. Furthermore, recent studies suggest that the action of Fringe modifies Notch to prevent it from interacting functionally with Serrate/Jagged ligands but allow it to preferentially bind Delta (Panin; Hicks). Although *Drosophila* has a single Fringe gene, vertebrates are known to express multiple genes (Radical, Manic and Lunatic Fringes) (Irvine).

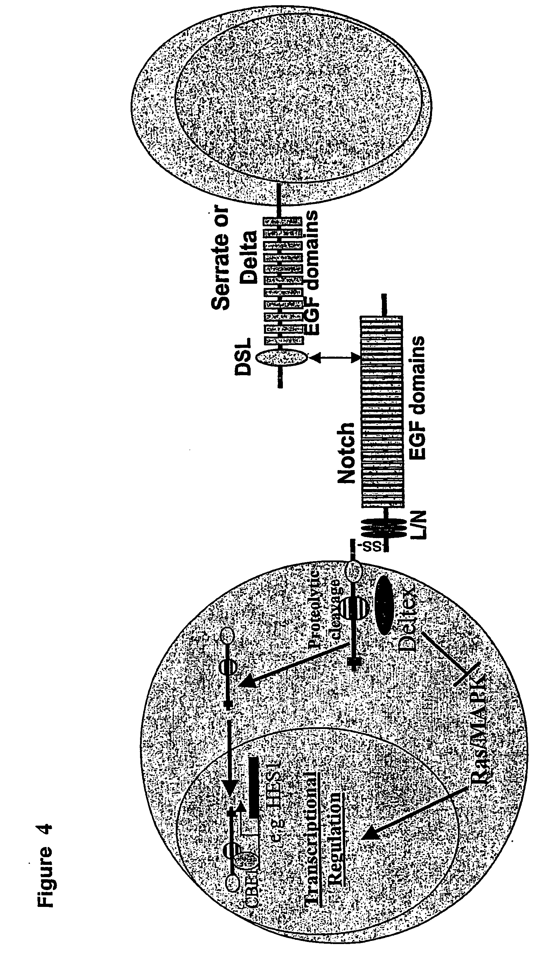

Signal transduction from the Notch receptor can occur via two different pathways (Fig 4). The better defined pathway involves proteolytic cleavage of the intracellular domain of Notch (Notch IC) that translocates to the nucleus and forms a transcriptional activator complex with the CSL family protein CBF1 (suppressor of Hairless, Su(H) in *Drosophila,* Lag-2 in *C. elegans).* NotchIC-CBF1 complexes then activate target genes, such as the bHLH proteins HES (hairy-enhancer of split like) 1 and 5. Notch can also signal in a CBF1-independent manner that invovles the sytoplasmic zinc finger containing protein Deltx (Fig 4). Unlike CBF1, Deltex does not move to the nucleus following Notch activation but instead can interact with Grb2 and modulate the Ras-INK signalling pathway.

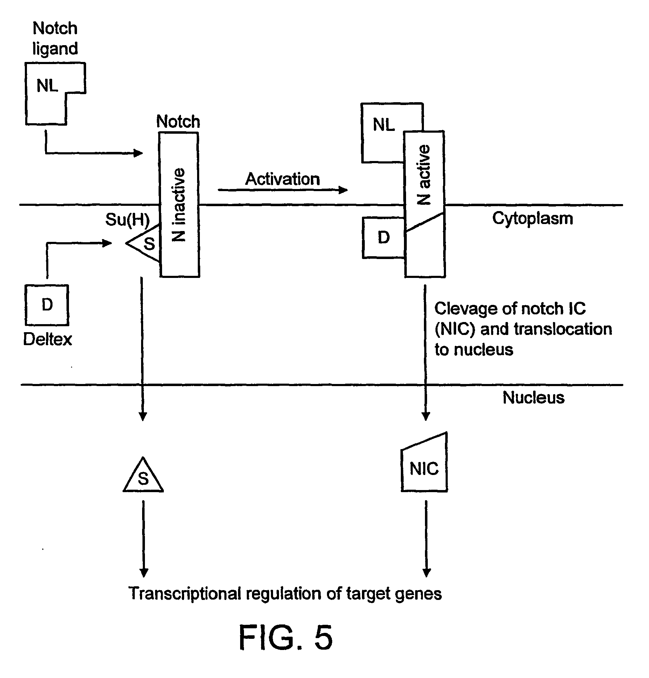

Thus, signal transduction from the Notch receptor can occur via two different pathways both of which are illustrated in Figure 4 and 5. Target genes of the Notch signalling pathway include Deltex, genes of the Hes family (Hes-1 in particular), Enhancer of Split [E(spl)] complex genes, IL-10, CD-23, CD-4 and Dll-1.

Deltex, an intracellular docking protein, replaces Su(H) as it leaves its site of interaction with the intracellular tail of Notch, as shown in Figure 3. Deltex is a cytoplasmic protein containing a zinc-finger (Artavanis-Tsakonas; Osborne). It interacts with the ankyrin repeats of the Notch intracellular domain. Studies indicate that Deltex promotes Notch pathway activation by interacting with Grb2 and modulating the Ras-JNK signalling pathway (Matsuno). Deltex also acts as a docking protein which prevents Su(H) from binding to the intracellular tail of Notch (Matsano). Thus, Su(H) is released into the nucleus where it acts as a transcriptional modulator. Recent evidence also suggests that, in a vertebrate B-cell system, Deltex, rather than the Su(H) homologue CBF1, is responsible for inhibiting E47 function (Ordentlich). Expression of Deltex is upregulated as a result of Notch activation in a positive feedback loop. The sequence of Homo sapiens Deltex (DTX1) mRNA may be found in GenBank Accession No. AF053700.

Hes-1 (Hump-enhancer of Split-1) (Takebayashi) is a transcriptional factor with a basic helix-loop-helix structure. It binds to an important functional site in the CD4 silencer leading to repression of CD4 gene expression. Thus, Hes-1 is strongly involved in the determination of T-cell fate. Other genes from the Hes family include Hes-5 (mammalian Enhancer of Split homologue), the expression of which is also upregulated by Notch activation, and Hes-3. Expression of Hes-1 is upregulated as a result of Notch activation. The sequence of Mus musculus Hes-1 can be found in GenBank Accession No. D16464.

The E(spl) gene complex [E(spl)-C] (Leimeister) comprises seven genes of which only E(spl) and Groucho show visible phenotypes when mutant. E(spl) was named after its ability to enhance Split mutations, Split being another name for Notch. Indeed, E(spl)-C genes repress Delta through regulation of achaete-scute complex gene expression. Expression of E(spl) is upregulated as a result of Notch activation.

IL-10 (interleukin-10) is a factor produced by Th2 helper T-cells. It is a co-regulator of mast cell growth and shows extensive homology with the Epstein-Barr berfi gene. Although it is not known to be a direct downstream target of the Notch signalling pathway, its expression has been found to be strongly upregulated coincident with Notch activation. The mRNA sequence of IL-10 may be found in GenBank ref. No. GI1041812.

CD-23 is the human leukocyte differentiation antigen CD23 (FCE2) which is a key molecule for B-cell activation and growth. It is the low-affinity receptor for IgE. Furthermore, the truncated molecule can be secreted, then functioning as a potent mitogenic growth factor. Although it is not thought to be a direct downstream target of the Notch signalling pathway, its expression has been found to be strongly upregulated coincident with Notch activation. The sequence for CD-23 may be found in GenBank ref No. GI1783344.

Dlx-1 (distalless-1) (McGuiness) expression is downregulated as a result of Notch activation. Sequences for Dlx genes may be found in GenBank Accession Nos. U51000-3.

CD-4 expression is downregulated as a result of Notch activation. A sequence for the CD-4 antigen may be found in GenBank Accession No. XM006966.

Other genes involved in the Notch signaling pathway, such as Numb, Mastermind and Dsh, and all genes the expression of which is modulated by Notch activation, are included in the scope of this invention.

### b. Polypeptides and Polynucleotides for Notch signaling Activation:

Examples of mammalian Notch ligands identified to date include the Delta family, for example Delta-1 (Genbank Accession No. AF003522 - *Homo sapiens),* Delta-3 (Genbank Accession No. AF084576 - *Rattus norvegicus)* and Delta-like 3 *(Mus musculus),* the Serrate family, for example Serrate-1 and Serrate-2 (WO97/01571, WO96/27610 and WO92/19734), Jagged-1 and Jagged-2 (Genbank Accession No. AF029778 - *Homo sapiens),* and LAG-2. Homology between family members is extensive. For example, human Jagged-2 has 40.6% identity and 58.7% similarity to Serrate.

Further homolognes of known mammalian Notch ligands may be identified using standard techniques. By a "homologue" it is meant a gene product that exhibits sequence homology, either amino acid or nucleic acid sequence homology, to any one of the known Notch ligands, for example as mentioned above. Typically, a homologue of a known Notch ligand will be at least 20%, preferably at least 30%, identical at the amino acid level to the corresponding known Notch ligand. Techniques and software for calculating sequence homology between two or more amino acid or nucleic acid sequences are well known in the art (see for example http:/www.ncbi.nlm.nih.gov and Ausubel et al., Current Protocols in Molecular Biology (1995), John Wiley & Sons, Inc.)

Notch ligands identified to date have a diagnostic DSL domain (D. *Delta,* S. *Serrate,* L. Lag2) comprising 20 to 22 amino acids at the amino terminus of the protein and between 3 to 8 EGF-like repeats on the extracellular surface. It is therefore preferred that homologues of Notch ligands also comprise a DSL domain at the N-terminus and between 3 to 8 EGF-like repeats on the extracellular surface.

Suitably the protein or polypeptide comprises a Notch ligand DSL an at least one EGF domain or a fragment, derivative, homologue, analogue or allelic variant thereof.

Preferably the protein for Notch activation comprises a Notch ligand DSL domain and at least 1 to 20, suitably at least 3 to 15, for example at least 3 to 8 Notch ligand EGF repeat motifs. Suitably the DSL and EGF sequences are or correspond to mammalian sequences. Preferred sequences include human sequences.

### Notch ligand Domains

As discussed above, Notch ligands comprise a number of distinctive domains. Some predicted/potential domain locations for various naturally occurring human Notch ligands (based on amino acid numbering in the precursor proteins) are shown below:

| **Human Delta 1** | | |
|---|---|---|
| **Component** | **Amino acids** | **Proposed function/domain** |
| SIGNAL | 1-17 | SIGNAL |
| CHAIN | 18-723 | DELTA-LIKE PROTEIN 1 |
| DOMAIN | 18-545 | EXTRACELLULAR |
| TRANSMEM | 546- 568 | TRANSMEMBRANE |
| DOMAIN | 569-723 | CYTOPLASMIC |
| DOMAIN | 159-221 | DSL |
| DOMAIN | 226-254 | EGF-LIKE 1 |
| DOMAIN | 257-285 | EGF-LIKE 2 |
| DOMAIN | 292-325 | EGF-LIKE 3 |
| DOMAIN | 332-363 | EGF-LIKE 4 |
| DOMAIN | 370-402 | EGF-LIKE 5 |
| DOMAIN | 409-440 | EGF-LIKE 6 |
| DOMAIN | 447-478 | EGF-LIKE 7 |
| DOMAIN | 485-516 | EGF-LIKE 8 |

| **Human Delta 3** | | |
|---|---|---|
| **Component** | **Amino acids** | **Proposed function/domain** |
| DOMAIN | 158-248 | DSL |
| DOMAIN | 278-309 | EGF-LIKE 1 |
| DOMAIN | 316-350 | EGF-LIKE 2 |
| DOMAIN | 357-388 | EGF-LIKE 3 |
| DOMAIN | 395-426 | EGF-LIKE 4 |
| DOMAIN | 433-464 | EGF-LIKE 5 |

| **Human Delta 4** | | |
|---|---|---|
| **Component** | **Amino acids** | **Proposed function/domain** |
| SIGNAL | 1-26 | SIGNAL |
| CHAIN | 27-685 | DELTA-LIKE PROTEIN 4 |
| DOMAIN | 27-529 | EXTRACELLULAR |
| TRANSMEM | 530-550 | TRANSMEMBRANE |
| DOMAIN | 551-685 | CYTOPLASMIC |
| DOMAIN | 155-217 | DSL |
| DOMAIN | 218-251 | EGF-LIKE 1 |
| DOMAIN | 252-282 | EGF-LIKE 2 |
| DOMAIN | 284-322 | EGF-LIKE 3 |
| DOMAIN | 324-360 | EGF-LIKE 4 |
| DOMAIN | 362-400 | EGF-LIKE 5 |
| DOMAIN | 402-438 | EGF-LlKE 6 |
| DOMAIN | 440-476 | EGF-LIKE 7 |
| DOMAIN | 480-518 | EGF-LIKE 8 |

| **Human Jagged 1** | | |
|---|---|---|
| **Component** | **Amino acids** | **Proposed function/domain** |
| SIGNAL | 1-33 | SIGNAL |
| CHAIN | 34-1218 | JAGGED 1 |
| DOMAIN | 34-1067 | EXTRACELLULAR |
| TRANSMEM | 1068-1093 | TRANSMEMBRANE |
| DOMAIN | 1094-1218 | CYTOPLASMIC |
| DOMAIN | 167-229 | DSL |
| DOMAIN | 234-262 | EGF-LIKE 1 |
| DOMAIN | 265-293 | EGF-LIKE 2 |
| DOMAIN | 300-333 | EGF-LIKE 3 |
| DOMAIN | 340-371 | EGF-LIKE 4 |
| DOMAIN | 378-409 | EGF-LIKE 5 |
| DOMAIN | 416-447 | EGF-LIKE 6 |
| DOMAIN | 454-484 | EGF-LIKE 7 |
| DOMAIN | 491-522 | EGF-LIKE 8 |
| DOMAIN | 529-560 | EGF-LIKE 9 |
| DOMAIN | 595-626 | EGF-LIKE 10 |
| DOMAIN | 633-664 | EGF-LIKE 11 |
| DOMAIN | 671-702 | EGF-LIKE 12 |
| DOMAIN | 709-740 | EGF-LIKE 13 |
| DOMAIN | 748-779 | EGF-LIKE 14 |
| DOMAIN | 786-817 | EGF-LIKE 15 |
| DOMAIN | 824-855 | EGF-LIKE 16 |
| DOMAIN | 863-917 | VON WILLEBRAND FACTOR C |

| **Human Jagged 2** | | |
|---|---|---|
| **Component** | **Amino acids** | **Proposed function/domain** |
| SIGNAL | 1-26 | SIGNAL |
| CHAIN | 27-1238 | JAGGED 2 |
| DOMAIN | 27-1080 | EXTRACELLULAR |
| TRANSMEM | 1081-1105 | TRANSMEMBRANE |
| DOMAIN | 1106-1238 | CYTOPLASMIC |
| DOMAIN | 178-240 | DSL |
| DOMAIN | 249-273 | EGF-LIKE 1 |
| DOMAIN | 276-304 | EGF-LIKE 2 |
| DOMAIN | 311-344 | EGF-LIKE 3 |
| DOMAIN | 351-382 | EGF-LIKE 4 |
| DOMAIN | 389-420 | EGF-LIKE 5 |
| DOMAIN | 427-458 | EGF-LIKE 6 |
| DOMAIN | 465-495 | EGF-LIKE 7 |
| DOMAIN | 502-533 | EGF-LIKE 8 |
| DOMAIN | 540-571 | EGF-LIKE 9 |
| DOMAIN | 602-633 | EGF-LIKE 10 |
| DOMAIN | 640-671 | EGF-LIKE 11 |
| DOMAIN | 678-709 | EGF-LIKE 12 |
| DOMAIN | 716-747 | EGF-LIKE 13 |
| DOMAIN | 755-786 | EGF-LIKE 14 |
| DOMAIN | 793-824 | EGF-LIKE 15 |
| DOMAIN | 831-862 | EGF-LIKE 16 |
| DOMAIN | 872-949 | VON WILLEBRAND FACTOR C |

### DSL domain

A typical DSL domain may include most or all of the following consensus amino acid sequence:

Cys Xaa Xaa Xaa Xaa Xaa Xaa Xaa Xaa Cys Xaa Xaa Xaa Cys Xaa Xaa
Xaa Xaa Xaa Xaa Xaa Xaa Xaa Xaa Xaa Cys Xaa Xaa Xaa Xaa Xaa Xaa
Xaa Cys Xaa Xaa Xaa Xaa Xaa Xaa Xaa Xaa Cys

Preferably the DSL domain may include most or all of the following consensus amino acid sequence: 
Cys Xaa Xaa Xaa ARO ARO Xaa Xaa Xaa Cys Xaa Xaa Xaa Cys BAS NOP
BAS ACM ACM Xaa ARO NOP ARO Xaa Xaa Cys Xaa Xaa Xaa NOP Xaa Xaa
Xaa Cys Xaa Xaa NOP ARO Xaa NOP Xaa Xaa Cys
 wherein:
ARO is an aromatic amino acid residue, such as tyrosine, phenylalanine, tryptophan or histidine;
NOP is a non-polar amino acid residue such as glycine, alanine, proline, leucine, isoleucine or valine;
BAS is a basic amino acid residue such as arginine or lysine; and
ACM is an acid or amide amino acid residue such as aspartic acid, glutamic acid, asparagine or glutamine.

Preferably the DSL domain may include most or all of the following consensus amino acid sequence: 
Cys Xaa Xaa Xaa Tyr Tyr Xaa Xaa Xaa Cys Xaa Xaa Xaa Cys Arg Pro
Arg Asx Asp Xaa Phe Gly His Xaa Xaa Cys Xaa Xaa Xaa Gly Xaa Xaa
Xaa Cys Xaa Xaa Gly Trp Xaa Gly Xaa Xaa Cys
 (wherein Xaa may be any amino acid and Asx is either aspartic acid or asparagine).

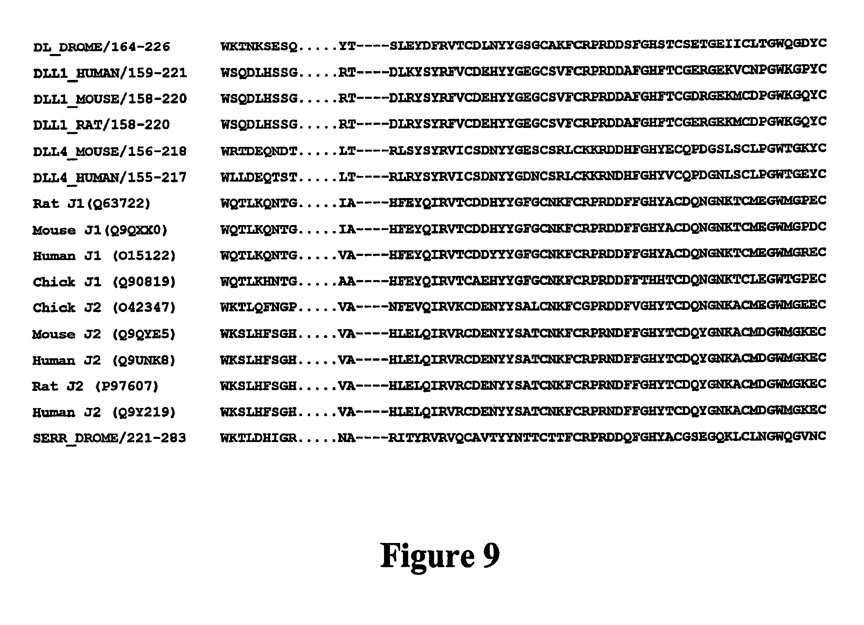

An alignment of DSL domains from Notch ligands from various sources is shown in Figure 9.

The DSL domain used may be derived from any suitable species, including for example Drosophila, Xenopus, rat, mouse or human. Preferably the DSL domain is derived from a vertebrate, preferably a mammalian, preferably a human Notch ligand sequence.

Suitably, for example, a DSL domain for use in the present invention may have at least 30%, preferably at least 50%, preferably at least 60%, preferably at least 70%, preferably at least 80%, preferably at least 90%, preferably at least 95% amino acid sequence identity to the DSL domain of human Jagged 1.

Alternatively a DSL domain for use in the present invention may, for example, have at least 30%, preferably at least 50%, preferably at least 60%, preferably at least 70%, preferably at least 80%, preferably at least 90%, preferably at least 95% amino acid sequence identity to the DSL domain of human Jagged 2.

Alternatively a DSL domain for use in the present invention may, for example, have at least 30%, preferably at least 50%, preferably at least 60%, preferably at least 70%, preferably at least 80%, preferably at least 90%, preferably at least 95% amino acid sequence identity to the DSL domain of human Delta 1.

Alternatively a DSL domain for use in the present invention may, for example, have at least 30%, preferably at least 50%, preferably at least 60%, preferably at least 70%, preferably at least 80%, preferably at least 90%, preferably at least 95% amino acid sequence identity to the DSL domain of human Delta 3.

Alternatively a DSL domain for use in the present invention may, for example, have at least 30%, preferably at least 50%, preferably at least 60%, preferably at least 70%, preferably at least 80%, preferably at least 90%, preferably at least 95% amino acid sequence identity to the DSL domain of human Delta 4.

### EGF like domain

The EGF-like motif has been found in a variety of proteins, as well as EGF and Notch and Notch ligands, including those involved in the blood clotting cascade (Furie and Furie, 1988, Cell 53: 505-518). For example, this motif has been found in extracellular proteins such as the blood clotting factors IX and X (Rees et aL, 1988, EMB4 J. 7:2053-2061; Furie and Furie, 1988, Cell 53: 505-518), in other Drosophila genes (Knust et aL, 1987 EMBO J. 761-766; Rothberg et aL, 1988, Cell 55:1047-1059), and in some cell-surface receptor proteins, such as thrombomodulin (Suzuki et al., 1987, EMBO J. 6:1891-1897) and LDL receptor (Sudhof et al., 1985, Science 228:815-822). A protein binding site has been mapped to the EGF repeat domain in thrombomodulin and urokinase (Kurosawa et al., 1988, J. Biol. Chem 263:5993-5996; Appella et al., 1987, J. BioL Chem. 262:4437-4440).

As reported by PROSITE the EGF domain typically includes six cysteine residues which have been shown (in EGF) to be involved in disulfide bonds. The main structure is proposed, but not necessarily required, to be a two-stranded beta-sheet followed by a loop to a C-terminal short two-stranded sheet. Subdomains between the conserved cysteines strongly vary in length as shown in the following schematic representation of the EGF-like domain: 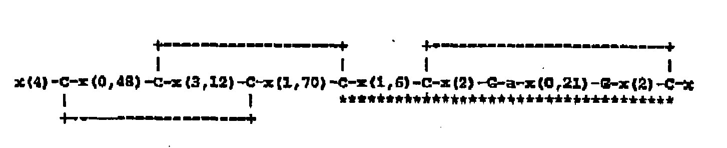 wherein:
'C': conserved cysteine involved in a disulfide bond.
'G': often conserved glycine
'a': often conserved aromatic amino acid
'*': position of both patterns.
'x': any residue

The region between the 5th and 6th cysteine contains two conserved glycines of which at least one is normally present in most EGF-like domains.

The EGF-like domain used may be derived from any suitable species, including for example Drosophila, Xenopus, rat, mouse or human. Preferably the EGF-like domain is derived from a vertebrate, preferably a mammalian, preferably a human Notch ligand sequence.

Suitably, for example, an EGF-like domain for use in the present invention may have at least 30%, preferably at least 50%, preferably at least 60%, preferably at least 70%, preferably at least 80%, preferably at least 90%, preferably at least 95% amino acid sequence identity to an EGF-like domain of human Jagged 1.

Alternatively an EGF-like domain for use in the present invention may, for example, have at least 30%, preferably at least 50%, preferably at least 60%, preferably at least 70%, preferably at least 80%, preferably at least 90%, preferably at least 95% amino acid sequence identity to an EGF-like domain of human Jagged 2.

Alternatively an EGF-like domain for use in the present invention may, for example, have at least 30%, preferably at least 50%, preferably at least 60%, preferably at least 70%, preferably at least 80%, preferably at least 90%, preferably at least 95% amino acid sequence identity to an EGF-like domain of human Delta 1.

Alternatively an EGF-like domain for use in the present invention may, for example, have at least 30%, preferably at least 50%, preferably at least 60%, preferably at least 70%, preferably at least 80%, preferably at least 90%, preferably at least 95% amino acid sequence identity to an EGF-like domain of human Delta 3.

Alternatively an EGF-like domain for use in the present invention may, for example, have at least 30%, preferably at least 50%, preferably at least 60%, preferably at least 70%, preferably at least 80%, preferably at least 90%, preferably at least 95% amino acid sequence identity to an EGF-like domain of human Delta 4.

It will be appreciated that whether or not any Notch ligand, homologue or combination of Notch Ligand domains is active may be determined by use of the an assay such as the assay described in Example 2 below.

In addition, suitable homologues of Notch ligands will be capable of binding to a Notch receptor. Binding may be assessed by a variety of techniques known in the art including *in vitro* binding assays.

Homologues of Notch ligands can be identified in a number of ways, for example by probing genomic or cDNA libraries with probes comprising all or part of a nucleic acid encoding a Notch ligand under conditions of medium to high stringency (for example 0.03M sodium chloride and 0.03M sodium citrate at from about 50°C to about 60°C). Alternatively, homologues may also be obtained using degenerate PCR which will generally use primers designed to target sequences within the variants and homologues encoding conserved amino acid sequences. The primers will contain one or more degenerate positions and will be used at stringency conditions lower than those used for cloning sequences with single sequence primers against known sequences.

Other substances capable of activating the Notch signalling pathway include compounds capable of upregulating Notch ligand expression including polypeptides that bind to and reduce or neutralise the activity of bone morphogenetic proteins (BMPs). Binding of extracellular BMPs (Wilson and Hemmati-Brivanlou, Hemmati-Brivanlou and Melton) to their receptors leads to down-regulated Delta transcription due to the inhibition of the expression of transcription factors of the achaete/scute complex. This complex is believed to be directly involved in the regulation of Delta expression. Thus, any substance that inhibits BMP expression and/or inhibits the binding of BMPs to their receptors may be capable of producing an increase in the expression of Notch ligands such as Delta and/or Serrate. Particular examples of such inhibitors include Noggin (Valenzuela), Chordin (Sasai), Follistatin (Iemura), Xnr3, and derivatives and variants thereof. Noggin and Chordin bind to BMPs thereby preventing activation of their signalling cascade which leads to decreased Delta transcription. Consequently, increasing Noggin and Chordin levels may lead to increase Notch ligand, in particular Delta, expression.

Furthermore, any substance that upregulates expression of transcription factors of the achaete/scute complex may also upregulate Notch ligand expression.

Other suitable substances that may be used to upregulate Notch ligand expression include transforming growth factors such as members of the fibroblast growth factor (FGF) family. The FGF may be a mammalian basic FGF, acidic FGF or another member of the FGF family such as an FGF-1, FGF-2, FGF-3, FGF-4, FGF-5, FGF-6, FGF-7. Preferably the FGF is not acidic FGF (FGF-1; Zhao). Most preferably, the FGF is a member of the FGF family which acts by stimulating the upregulation of expression of a Serrate polypeptide on APCs. The inventors have shown that members of the FGF family can upregulate Serrate-1 gene expression in APCs.

Immunosuppressive cytokines may also be used to upregulate Notch ligand expression. Examples include members of the TGF-β family such as TGF-β-1 and TGF-β-2, and interleukins such as IL-4, IL-10 and IL-13, and FLT3 ligand. The TGF-β family can upregulate Notch, particularly Notch 1, expression; IL-10 can upregulate Serrate, particularly Senate 1, expression; IL-10 can upregulate Notch, Delta and Serrate, particularly Notch 2, Notch 4, Delta 1 and Serrate 1, expression; and IL-10 can upregulate Serrate, particularly Serrate 1, expression.

The substance capable of upregulating expression of Notch or a Notch ligand may be selected from polypeptides and fragments thereof, linear peptides, cyclic peptides, including synthetic and natural compounds. The substances capable of upregulating expression of a Notch ligand may be derived from a biological material such as a component of extracellular matrix. Suitable extracellular matrix components are derived from immunologically privileged sites such as the eye. For example aqueous humour or components thereof may be used.

Polypeptide substances such as Noggin, FGFs and TGF-β may be purified from mammalian cells, obtained by recombinant expression in suitable host cells or obtained commercially. Alternatively, nucleic acid constructs encoding the polypeptides may be used. As a further example, overexpression of Notch or Notch ligand, such as Delta or Serrate, may be brought about by introduction of a nucleic acid construct capable of activating the endogenous gene, such as the Serrate or Delta gene. In particular, gene activation can be achieved by the use of homologous recombination to insert a heterologous promoter in place of the natural promoter, such as the Serrate or Delta promoter, in the genome of the target cell.

The activating molecule of the present invention will, in an alternative embodiment, be capable of modifying Notch-protein expression or presentation on the cell membrane or signalling pathways. Agents that enhance the presentation of a fully functional Notch-protein on the target cell surface include matrix metalloproteinases such as the product of the Kuzbanian gene of Drosophila (Dkuz) and other ADAMALYSIN gene family members.

Activators of the Notch signalling pathway also include antibodies to the aforementioned activators.

### c. Polypeptides and Polynucleotides for Notch signalling Inhibition

Substances that may be used to inhibit Notch ligand expression include nucleic acid sequences encoding polypeptides that affect the expression of genes encoding Notch ligands. For instance, for Delta expression, binding of extracellular BMPs (bone morphogenetic proteins, Wilson and Hemmati-Brivanlou; Hemmati-Brivanlou and Melton) to their receptors leads to down-regulated Delta transcription due to the inhibition of the expression of transcription factors of the achaete/scute complex. This complex is believed to be directly involved in the regulation of Delta expression. Thus, any polypeptide that upregulates BMP expression and/or stimulates the binding of BMPs to their receptors may be capable of producing a decrease in the expression of Notch ligands such as Delta and/or Serrate. Examples may include nucleic acids encoding BMPs themselves. Furthermore, any substance that inhibits expression of transcription factors of the achaete/scute complex may also downregulate Notch ligand expression.

Members of the BMP family include BMP1 to BMP6, BMP7 also called OP1, OP2 (BMP8) and others. BMPs belong to the transforming growth factor beta (TGF-beta) superfamily, which includes, in addition to the TGF-betas, activins/inhibins (e.g., alpba-inhibin), mullerian inhibiting substance, and glial cell line-derived neurotrophic factor.

Other examples of polypeptides that inhibit the expression of Delta and/or Serrate include the Toll-like receptor (Medzhitov) or any other receptors linked to the innate immune system (for example CD14, complement receptors, scavenger receptors or defensin proteins), and other polypeptides that decrease or interfere with the production of Noggin (Valenzuela), Chordin (Sasai), Follistatin (Iemura), Xnr3, and derivatives and variants thereof. Noggin and Chordin bind to BMPs thereby preventing activation of their signalling cascade which leads to decreased Delta transcription. Consequently, reducing Noggin and Chordin levels may lead to decrease Notch ligand, in particular Delta, expression.

In more detail, in Drosophila, the Toll transmembrane receptor plays a central role in the signalling pathways that control amongst other things the innate nonspecific immune response. This Toll-mediated immune response reflects an ancestral conserved signalling system that has homologous components in a wide range of organisms. Human Toll homologues have been identified amongst the Toll-like receptor (TLR) genes and Toll/interleukin-1 receptor-like (TIL) genes and contain the characteristic Toll motifs: an extracellular leucine-rich repeat domain and a cytoplasmic interleukin-1 receptor-like region. The Toll-like receptor genes (including TIL genes) now include TLR4, TIL3, TIL4, and 4 other identified TLR genes.

Other suitable sequences that may be used to downregulate Notch ligand expression include those encoding immune costimulatory molecules (for example CD80, CD86, ICOS, SLAM) and other accessory molecules that are associated with immune potentiation (for example CD2, LFA-1).

Other suitable substances that may be used to downregulate Notch ligand expression include nucleic acids that inhibit the effect of transforming growth factors such as members of the fibroblast growth factor (FGF) family. The FGF may be a mammalian basic FGF, acidic FGF or another member of the FGF family such as an FGF-1, FGF-2, FGF-3, FGF-4, FGF-5, FGF-6, FGF-7. Preferably the FGF is not acidic FGF (FGF-1; Zhao *et al.,* 1995). Most preferably, the FGF is a member of the FGF family which acts by stimulating the upregulation of expression of a Senate polypeptide on APCs. The inventors have shown that members of the FGF family can upregulate Serrate-1 gene expression in APCs.

Suitable nucleic acid sequences may include ami-sense constructs, for example nucleic acid sequences encoding antisense Notch ligand constructs as well as antisense constructs designed to reduce or inhibit the expression of upregulators of Notch ligand expression (see above). The antisense nucleic acid may be an oligonucleotide such as a synthetic single-stranded DNA. However, more preferably, the antisense is an antisense RNA produced in the patient's own cells as a result of introduction of a genetic vector. The vector is responsible for production of antisense RNA of the desired specificity on introduction of the vector into a host cell.

Preferably, the nucleic acid sequence for use in the present invention is capable of inhibiting Serrate and Delta, preferably Serrate 1 and Serrate 2 as well as Delta 1 and Delta 3 expression in APCs such as dendritic cells. In particular, the nucleic acid sequence may be capable of inhibiting Serrate expression but not Delta expression in APCs. Alternatively, the nucleic acid sequence for use in the present invention is capable of inhibiting Delta expression in T cells such as CD4⁺ helper T cells or other cells of the immune system that express Delta (for example in response to stimulation of cell surface receptors). In particular, the nucleic acid sequence may be capable of inhibiting Delta expression but not Serrate expression in T cells. In a particularly preferred embodiment, the nucleic acid sequence is capable of inhibiting Notch ligand expression in both T cells and APC, for example Serrate expression in APCs and Delta expression in T cells.

Preferred suitable substances that may be used to downregulate Notch ligand expression include growth factors and cytokines. More preferably soluble protein growth factors may be used to inhibit Notch or Notch ligand expression. For instance, Notch ligand expression may be reduced or inhibited by the addition of BMPs or activins (a member of the TGF-β superfamily). In addition, T cells, APCs or tumour cells could be cultured in the presence of inflammatory type cytokines including IL-12, IFN-γ, IL-18, TNF-α, either alone or in combination with BMPs.

Molecules for inhibition of Notch signalling will also include polypeptides, or polynucleotides which encode therefore, capable of modifying Notch-protein expression or presentation on the cell membrane or signalling pathways. Molecules that reduce or interfere with its presentation as a fully functional cell membrane protein may include MMP inhibitors such as hydroxymate-based inhibitors.

Other substances which may be used to reduce interaction between Notch and Notch ligands are exogenous Notch or Notch ligands or functional derivatives thereof. Such Notch ligand derivatives would preferably have the DSL domain at the N-terminus and between 3 to S EGF-like repeats on the extracellular surface. A peptide corresponding to the Delta/Serrate/LAG-2 domain of hJagged1 and supernatants from COS cells expressing a soluble form of the extracellular portion of hJagged1 was found to mimic the effect of Jagged1 in inhibiting Notch1 (Li).

Other Notch signalling pathway antagonists include antibodies which inhibit interactions between components of the Notch signalliing pathway, e.g. antibodies to Notch ligands.

Whether a substance can be used for modulating Notch-Notch ligand expression may be determined using suitable screening assays.

Notch signalling can be monitored either through protein assays or through nucleic acid assays. Activation of the Notch receptor leads to the proteolytic cleavage of its cytoplasmic domain and the translocation thereof into the cell nucleus. The "detectable signal" referred to herein may be any detectable manifestation attributable to the presence of the cleaved intracellular domain of Notch. Thus, increased Notch signalling can be assessed at the protein level by measuring intracellular concentrations of the cleaved Notch domain. Activation of the Notch receptor also catalyses a series of downstream reactions leading to changes in the levels of expression of certain well defined genes. Thus, increased Notch signalling can be assessed at the nucleic acid level by say measuring intracellular concentrations of specific mRNAs. In one preferred embodiment of the present invention, the assay is a protein assay. In another preferred embodiment of the present invention, the assay is a nucleic acid assay.

The advantage of using a nucleic acid assay is that they are sensitive and that small samples can be analysed.

The intracellular concentration of a particular mRNA, measured at any given time, reflects the level of expression of the corresponding gene at that time. Thus, levels of mRNA of downstream target genes of the Notch signalling pathway can be measured in an indirect assay of the T-cells of the immune system. In particular, an increase in levels of Deltex, Hes-1 and/or IL-10 mRNA may, for instance, indicate induced anergy while an increase in levels of DII-1 or IFN-(mRNA, or in the levels of mRNA encoding cytokines such as IL-2, IL-5 and IL-13, may indicate improved responsiveness.

Various nucleic acid assays are known. Any convention technique which is known or which is subsequently disclosed may be employed. Examples of suitable nucleic acid assay are mentioned below and include amplification, PCR, RT-PCR, RNase protection, blotting, spectrometry, reporter gene assays, gene chip arrays and other hybridization methods.

In particular, gene presence, amplification and/or expression may be measured in a sample directly, for example, by conventional Southern blotting, Northern blotting to quantitate the transcription of mRNA, dot blotting (DNA or RNA analysis), or in situ hybridisation, using an appropriately labelled probe. Those skilled in the art will readily envisage how these methods may be modified, if desired.

PCR was originally developed as a means of amplifying DNA from an impure sample. The technique is based on a temperature cycle which repeatedly heats and cools the reaction solution allowing primers to anneal to target sequences and extension of those primers for the formation of duplicate daughter strands. RT-PCR uses an RNA template for generation of a first strand cDNA with a reverse transcriptase. The cDNA is then amplified according to standard PCR protocol. Repeated cycles of synthesis and denaturation result in an exponential increase in the number of copies of the target DNA produced. However, as reaction components become limiting, the rate of amplification decreases until a plateau is reached and there is little or no net increase in PCR product. The higher the starting copy number of the nucleic acid target, the sooner this "end-point" is reached.

Real-time PCR uses probes labeled with a fluorescent tag or fluorescent dyes and differs from end-point PCR for quantitative assays in that it is used to detect PCR products as they accumulate rather than for the measurement of product accumulation after a fixed number of cycles. The reactions are characterized by the point in time during cycling when amplification of a target sequence is first detected through a significant increase in fluorescence.

The ribonuclease protection (RNase protection) assay is an extremely sensitive technique for the quantitation of specific RNAs in solution . The ribonuclease protection assay can be performed on total cellular RNA or poly(A)-selected mRNA as a target. The sensitivity of the ribonuclease protection assay derives from the use of a complementary *in vitro* transcript probe which is radiolabeled to high specific activity.
The probe and target RNA are hybridized in solution, after which the mixture is diluted and treated with ribonuclease (RNase) to degrade all remaining single-stranded RNA. The hybridized portion of the probe will be protected from digestion and can be visualized via electrophoresis of the mixture on a denaturing polyacrylamide gel followed by autoradiography. Since the protected fragments are analyzed by high resolution polyacrylamide gel electrophoresis, the ribonuclease protection assay can be employed to accurately map mRNA features. If the probe is hybridized at a molar excess with respect to the target RNA, then the resulting signal will be directly proportional to the amount of complementary RNA in the sample.

Gene expression may also be detected using a reporter system. Such a reporter system may comprise a readily identifiable marker under the control of an expression system, e.g. of the gene being monitored. Fluorescent markers, which can be detected and sorted by FACS, are preferred. Especially preferred are GFP and luciferase. Another type of preferred reporter is cell surface markers, i.e. proteins expressed on the cell surface and therefore easily identifiable.

In general, reporter constructs useful for detecting Notch signalling by expression of a reporter gene may be constructed according to the general teaching of Sambrook et al (1989). Typically, constructs according to the invention comprise a promoter by the gene of interest, and a coding sequence encoding the desired reporter constructs, for example of GFP or luciferase. Vectors encoding GFP and luciferase are known in the art and available commercially.

Sorting of cells, based upon detection of expression of genes, may be performed by any technique known in the art, as exemplified above. For example, cells may be sorted by flow cytometry or FACS. For a general reference, see Flow Cytometry and Cell Sorting: A Laboratory Manual (1992) A. Radbruch (Ed.), Springer Laboratory, New York.

Flow cytometry is a powerful method for studying and purifying cells. It has found wide application, particularly in immunology and cell biology: however, the capabilities of the FACS can be applied in many other fields of biology. The acronym F.A.C.S. stands for Fluorescence Activated Cell Sorting, and is used interchangeably with "flow cytometry". The principle of FACS is that individual cells, held in a thin stream of fluid, are passed through one or more laser beams, causing light to be scattered and fluorescent dyes to emit light at various frequencies. Photomultiplier tubes (PMT) convert light to electrical signals, which are interpreted by software to generate data about the cells. Sub-populations of cells with defined characteristics can be identified and automatically sorted from the suspension at very high purity (~100%).

FACS can be used to measure gene expression in cells transfected with recombinant DNA encoding polypeptides. This can be achieved directly, by labelling of the protein product, or indirectly by using a reporter gene in the construct. Examples of reporter genes are β-galactosidase and Green Fluorescent Protein (GFP). β-galactosidase activity can be detected by FACS using fluorogenic substrates such as fluorescein digalactoside (FDG). FDG is introduced into cells by hypotonic shock, and is cleaved by the enzyme to generate a fluorescent product, which is trapped within the cell. One enzyme can therefore generate a large amount of fluorescent product. Cells expressing GFP constructs will fluoresce without the addition of a substrate. Mutants of GFP are available which have different excitation frequencies, but which emit fluorescence in the same channel. In a two-laser FACS machine, it is possible to distinguish cells which are excited by the different lasers and therefore assay two transfections at the same time.

Alternative means of cell sorting may also be employed. For example, the invention comprises the use of nucleic acid probes complementary to mRNA. Such probes can be used to identify cells expressing polypeptides individually, such that they may subsequently be sorted either manually, or using FACS sorting. Nucleic acid probes complementary to mRNA may be prepared according to the teaching set forth above, using the general procedures as described by Sambrook et al (1989).

In a preferred embodiment, the invention comprises the use of an antisense nucleic acid molecule, complementary to a mRNA, conjugated to a fluorophore which may be used in FACS cell sorting.

Methods have also been described for obtaining information about gene expression and identity using so-called gene chip arrays or high density DNA arrays (Chee). These high density arrays are particularly useful for diagnostic and prognostic purposes. Use may also be made of In Vivo Expression Technology (IVET) (Camilli). IVET identifies genes up-regulated during say treatment or disease when compared to laboratory culture.

The advantage of using a protein assay is that Notch activation can be directly measured. Assay techniques that can be used to determine levels of a polypeptide are well known to those skilled in the art. Such assay methods include radioimmunoassays, competitive-binding assays, Western Blot analysis, antibody sandwich assays, antibody detection, FACS and ELISA assays.

### Preparation of Primed APCs and Lymphocytes

According to one aspect of the invention immune cells may be used to present antigens or allergens and/or may be treated to modulate expression or interaction of Notch, a Notch ligand or the Notch signalling pathway. Thus, for example, Antigen Presenting Cells (APCs) may be cultured in a suitable culture medium such as DMEM or other defined media, optionally in the presence of a serum such as fetal calf serum. Optimum cytokine concentrations may be determined by titration. One or more substances capable of up-regulating or down-regulating the Notch signalling pathway are then typically added to the culture medium together with the antigen of interest. The antigen may be added before, after or at substantially the same time as the substance(s). Cells are typically incubated with the substance(s) and antigen for at least one hour, preferably at least 3 hours, if necessary for at least 12 hours or more at 37°C. If required, a small aliquot of cells may be tested for modulated target gene expression as described above. Alternatively, cell activity may be measured by the inhibition of T cell activation by monitoring surface markers, cytokine secretion or proliferation as described in WO98/20142. APCs transfected with a nucleic acid construct directing the expression of, for example Serrate, may be used as a control.

As discussed above, polypeptide substances may be administered to APCs by introducing nucleic acid constructs/viral vectors encoding the polypeptide into cells under conditions that allow for expression of the polypeptide in the APC. Similarly, nucleic acid constructs encoding antigens may be introduced into the APCs by transfection, viral infection or viral transduction. The resulting APCs that show increased levels of a Notch signalling are now ready for use.

The techniques described below are described in relation to T cells, but are equally applicable to B cells. The techniques employed are essentially identical to that described for APCs alone except that T cells are generally co-cultured with the APCs. However, it may be preferred to prepare primed APCs first and then incubate them with T cells. For example, once the primed APCs have been prepared, they may be pelleted and washed with PBS before being resuspended in fresh culture medium. This has the advantage that if, for example, it is desired to treat the T cells with a different substance(s) capable of modulating presemilin to that used with the APC, then the T cell will not be brought into contact with the different substance(s) used in the APC. Alternatively, the T cell may be incubated with a first substance (or set of substances) to modulate presenilin or presenilin-dependent gamma-secretase and, optionally, Notch signalling, washed, resuspended and then incubated with the primed APC in the absence of both the substance(s) used to modulate the APC and the substance(s) used to modulate the T cell. Alternatively, T cells may be cultured and primed in the absence of APCs by use of APC substitutes such as anti-TCR antibodies (e.g, anti-CD3) with or without antibodies to costimulatory molecules (e.g. anti-CD28) or alternatively T cells may be activated with MHC-peptide complexes (e.g. tetramers).

Incubations will typically be for at least 1 hour, preferably at least 3 or 6 hours, in suitable culture medium at 37°C. Induction of immunotolerance may be determined by subsequently challenging T cells with antigen and measuring IL-2 production compared with control cells not exposed to APCs.

T cells or B cells which have been primed in this way may be used according to the invention to induce immunotolerance in other T cells or B cells.

### Example 1

A fusion protein was made between the N-tetminal 90 amino acids of TSST1 and an N-terminal fragment of human Jagged1 such that the Jagged1 fragment was at the N-terminus of the fusion protein and the TSST1 fragment at the C-terminus.

A fragment of human Jagged 1 (hJag1) cDNA (see for example GenBank Accession No U61276) coding for the sequence from amino acid 1 of the immature protein sequence (i.e. the Met (M) residue used to initiate transcription) through to amino acid 296 (Asp (D)) was prepared in a pcDNA3.1 expression vector (Invitrogen, Carlsbad, CA, USA and Paisley, UK). The nucleic acid sequence of the Jagged 1 cDNA fragment was as follows: 
atgcgttccccacggacgcgcggccggtccgggcgccccctaagcctcctgctcgccctgctctgtgcc
ctgcgagccaaggtgtgtggggcctcgggtcagttcgagttggagatcctgtccatgcagaacgtgaac
ggggagctgcagaacgggaactgctgcggcggcgcccggaacccgggagaccgcaagtgcacccgcgac
gagtgtgacacatacttcaaagtgtgcctcaaggagtatcagtcccgcgtcacggccgggggccctgc
agcttcggctcagggtccacgcctgtcatcgggggcaacaccttcaacctcaaggccagccgcggcaac
gaccgcaaccgcatgctgcctttcagtttcgcctggccgaggtcctatacgttgcttgtggaggcgtgg
gattccagtaatgacaccgttcaacctgacagtattattgaaaaggcttctcactcgggcatgatcaac
cccagccggcagtggcagacgctgaagcagaacacgggcgttgcccactttgagtatcagatccgcgtg
acctgtgatgactactactatggctttggctgcaataagttctgccgccccagagatgacttctttgga
 
cactatgcctgtgaccagaatggcaacaaacttgcatggaaggctggatggccccgaatgtaacaga
gctatttgccgacaaggctgcagtcctaagcatgggtcttgcaaactcccaggtgactgcaggtgccag
tatggctggcaaggcctgtactgtgataagtgcatcccacacccgggatgcgtccacggcatctgtaat
gagccctggcagtgcctctgtgagaccaactggggcggccagctctgtgacaaagatct
 Amino acid 296 naturally forms part of the recognition sequence of the restriction site BglII (AGATCT), and therefore the TSST-1 fragment site was cloned as a BglII/EcoRI piece in frame into the hJagged1 vector using the BglII site to generate an open reading frame coding for the following:
hJag1 amino acids 1-296; (Gly-Ser)₅ artificial linker; TSST-1 N-terminal sequence amino acids 1-90.

The hJag1 296 amino acid fragment in the resulting fusion protein had the following amino acid sequence:

1   MRSPRTRGRS GRPLSLLLAL LCALRAKVCG ASGQFELEIL SMQNVNGELQ NGNCCGGARN
61  PGDRKCTRDE CDTYFKVCLK EYQSRVTAGG PCSFGSGSTP VIGGNTFNLK ASRGNDRNRI
121 VLPFSFAWPR SYTLLVEAWD SSNDTVQPDS IIEKASHSGM INPSRQWQTL KQNTGVAHFE
181 YQIRVTCDDY YYGFGCNKFC RPRDDFFGHY ACDQNGNKTC MEGWMGPECN RAICRQGCSP
241 KHGSCKLPGD CRCQYGWQGL YCDKCIPHPG CVHGICNEPW QCLCETNWGG QLCDKD

The TSST-1 N-terminal 90 amino acid fragment in the resulting fusion protein had the following amino acid sequence:

1 STNDNIKDLL DWYSSGSDTF TNSEVLDNSL GSMRIKNTDG SISLIIFPSP YYSPAFTKGE
61 KVDLNTKRTK KSQHTSEGTY IHFQISGVTN

The (Gly-Ser)₅ artificial linker sequence in the resulting fusion protein had the amino acid sequence: GSGSGSGSGS

To provide the TSST/linker portion of the fusion protein, a polynucleotide coding TSST-1 N-terminal sequence amino acids 1-90 and the 5' (Gly-Ser)₅ linker was generated with the following nucleic acid sequence:

gat ctc ggc tct ggt agc gga agt ggc agc ggc tct agt act aac gac aac atc aag gat ctg ctt gac
tgg tac tct tcc ggg tcg gat aca ttt acg aat agc gaa gta tta gat aat tca cta ggc tca atg aga
ata aaa aac acc gac ggc tcc ata agt ctc atc att ttt cca agt cca tat tat tcg cca gca ttc aca
aaa ggt gaa aaa gta gat ttg aat aca aag aga act aaa aag tct caa cac acc agt gag gga acg
tac ata cat ttc cag att agc gga gta aca aat tga (stop)

This polynucleotide was generated by gene assembly (using oligonucleotide primers followed by PCR amplification using oligonucleotide primers designed to amplify from the 5' and 3' ends at the same time as introducing BglII and EcoRI sites respectively), by annealing overlapping "upper strand" and "lower strand" primers (selected such that the upper and lower strand primers overlap with each other by approximately 12-15 base pairs) as follows:
upper strand primers: Gm40, 42, 44, 46 and 48: 
Gm40: ata aga atc aga tct cgg ctc tgg tag cgg aag tgg cag cgg ctc tag tac t
Gm42: gat ctg ctt gac tgg tac tct tcc ggg tcg gat aca ttt acg aat agc
Gm44: ggc tca atg aga ata aaa aac acc gac ggc tcc ata agt ctc atc att ttt
Gm46: gca ttc aca aaa ggt gaa aaa gta gat ttg aat aca aag aga act aaa aag
Gm48: acg tac ata cat ttc cag att agc gga gta aca aat tga gaa ttc ata aga atg
lower strand primers: Gm41, 43, 45, 47 and 50: 
Gm41: cca gtc aag cag atc ctt gat gtt gtc gtt agt act aga gcc gct gcc act
Gm43: tat tct cat tga gcc tag tga att atc taa tac ttc gct att cgt aaa tgt
Gm45: acc ttt tgt gaa tgc tgg cga ata ata tgg act tgg aaa aat gat gag act tat
Gm47: gaa atg tat gta cgt tcc ctc act ggt gtg ttg aga ctt ttt agt tct ctt tgt
Gm50: cat tct tat gaa ttc tc
Annealing and PCR were carried out as follows:
   1. All upper strand primers were mixed together in equal quantities. All lower strand primers were mixed together in equal quantities.
   2. A primary PCR reaction was set up using 1ul of a 2:1 upper:lower primer mix as target and Pfu DNA polymerase using the fallowing cycling conditions on an MJ Tetrad ^{™}PCR machine:
      40°C for 2'; 72°C for 30 seconds; then 30 cycles of 94°C for 30 seconds, 45°C for 30 seconds, 72°C for 30 seconds; followed by a 16°C soak.
      This reaction yields a mixture of products but a significant proportion of this product runs at the predicted size of 330bp.
   3. The 330bp product was gel-purified using Qiaquick^{™} Gel Extraction kit and used as target in a secondary PCR using primers Gm49 (ata aga ate aga tct cgg ctc) and Gm50 (described above). Pfu DNA polymerase was used with the following cycling conditions on an MJ Tetrad PCR machine:
      94°C for 1'; then 30 cycles of 94°C for 30 seconds, 49°C for 30 seconds, 72°C for 1'; followed by 72°C for 10' and a 16°C soak.
      This generated a single product of 330bp (note that gm49 and gm50 contain the BglII and EcoRI sites, respectively, used to clone out the final fragment).
   4. The product was gel-purified as above and then digested with BglII and EcoRI, and was then cloned into the expression vector already containing the 5' end of the hJag1 sequence.

C2C12 cells were then transiently transfected (using Effectene^{™} transfection reagent; Qiagen, Valencia, CA, US and Crawley, UK)-with 0.4µg of the resulting pcDNA3.1 expression vector DNA and the transfected cells were cultured to express the final fusion protein.

### Reference Example 2

### CHO-N2 (N27) Luciferase Reporter Assay

### A) Construction of Luciferase Reporter Plasmid 10xCBF1-Luc (pLOR91)

An adenovirus major late promoter TATA-box motif with BglII and HindIII cohesive ends was generated as follows:

BglII HindIII
GATCTGGGGGCTATAAAAGGGGGTA
ACCCCCGATATTTTCCCCCATTCGA

This was cloned into plasmid pGL3-Basic (Promega) between the BgiII and HindIII sites to generate plasmid pGL3-AdTATA.

A TP1 promoter sequence (TP1; equivalent to 2 CBF1 -repeats) with BamHI and BglII cohesive ends was generated as follows:

BamH1                                                    BglII
5' GATCCCGACTCGTGGGAAAATGGCGGAAGGCACCGTGGGAAAATAGTA 3'
   3'      GGCTGAGCACCCTTTTACCGCCTTCCCGTGGCACCCTTTTATCATCTAG 5'

This sequence was pentamerised by repeated insertion into a BglII site and the resulting TP1 pentamer (equivalent to 10 CBF1 repeats) was inserted into pGL3. AdTATA at the BglII site to generate plasmid pLOR91.

### B) Generation of a stable CHO cell reporter cell line expressing full length Notch2 and the 10xCBF1-Luc reporter cassette

A cDNA clone spanning the complete coding sequence of the human Notch2 gene (see, eg GenBank Accession No AF315356) was constructed as follows. A 3' cDNA fragment encoding the entire intracellular domain and a portion of the extracellular domain was isolated from a human placental cDNA library (OriGene Technologies Ltd., USA) using a PCR-based screening strategy. The remaining 5' coding sequence was isolated using a RACE (Rapid Amplification of cDNA Ends) strategy and ligated onto the existing 3' fragment using a unique restriction site common to both fragments (Cla I). The resulting full-length cDNA was then cloned into the mammalian expression vector pcDNA3.1-V5-HisA (Invitrogen) without a stop codon to generate plasmid pLOR92. When expressed in mammalian cells, pLOR92 thus expresses the full-length human Notch2 protein with V5 and His tags at the 3' end of the intracellular domain.

Wild-type CHO-K1 cells (eg see ATCC No CCL 61) were transfected with pLOR92 (pcDNA3.1-FLNotch2-V5-His) using Lipfectamine 2000^{™} (Invitrogen) to generate a stable CHO cell clone expressing full length human Notch2 (N2). Transfectant clones were selected in Dulbecco's Modified Eagle Medium (DMEM) plus 10% heat inactivated fetal calf serum ((HI)FCS) plus glutamine plus Penicillin-Streptomycin (P/S) plus 1 mg/ml G418 (Geneticin^{™}- Invitrogen) in 96-well plates using limiting dilution Individual colonies were expanded in DMEM plus 10%(HI)FCS plus glutamine plus PIS plus 0.5 mg/ml G418. Clones were tested for expression of N2 by Western blots of cell lysates using an anti-V5 monoclonal antibody (Invitrogen). Positive clones were then tested by transient transfection with me reporter vector pLOR91 (10xCBF1-Luc) and co-culture with a stable CHO cell clone (CHO-Delta) expressing full length human delta-like ligand 1 (DLL1; eg see GenBank Accession No AF196571). (CHO-Delta was prepared in the same way as the CHO Notch 2 clone, but with human DLL1 used in place of Notch 2. A strongly positive clone was selected by Western blots of cell lysates with anti-V5 mAb.)

One CHO-N2 stable clone, N27, was found to give high levels of induction when transiently transfected with pLOR91 (10xCBF1-Luc) and co-cultured with the stable CHO cell clone expressing full length human DLL1 (CHO-Delta1). A hygromycin gene cassette (obtainable from pcDNA3.1/hygro, Invitrogen) was inserted into pLOR91 (10xCBF1-Luc) using BamH1 and Sal1 and this vector (10xCBF1-Luc-hygro) was transfected into the CHO-N2 stable clone (N27) using Lipfectamine 2000 (Invitrogen). Transfectant clones were selected in DMEM plus 10%(HI)FCS plus glutamine plus PIS plus 0.4 mg/ml hygromycin B (Invitrogen) plus 0.5 mg/ml G418 (Invitrogen) in 96-well plates using limiting dilution. Individual colonies were expanded in DMEM plus 10%(HI)FCS plus glutamine plus PIS + 0.2 mg/ml hygromycin B plus 0.5 mg/ml G418 (Invitrogen).

Clones were tested by co-culture with a stable CHO cell clone expressing FL human DLL1. Three stable reporter cell lines were produced N27#11, N27#17 and N27#36. N27#11 was selected for further use because of its low background signal in the absence of Notch signalling, and hence high fold induction when signalling is initiated. Assays were set up in 96-well plates with 2 x 10⁴ N27#11 cells per well in 100 µl per well of DMEM plus 10%(HI)FCS plus glutamine plus P/S.

### C) Transient Transfection of CHO-N2 Cells with 10xcBF1-Luc

Alternatively, for transient transfection, CHO-N2 (Clone N27) cells were maintained in DMEM plus 10%(HI)FCS plus glutamine plus P/S plus 0.5 mg/ml G418 and a T₈₀ flask of the CHO-N2 cells was transfected as follows. The medium on the cells was replaced with 8 ml of fresh in DMEM plus 10%(HI)FCS plus glutamine plus P/S. In a sterile bijou 10 µg of pLOR91 (10xCBF1-Luc) was added to OptiMem (Invitrogen) to give a final volume of 1 ml and mixed. In a second sterile bijou 20 µl of Lipofectamine 2000 reagent was added to 980 µl of OptiMem and mixed.

The contents of each bijou were mixed and left at room temperature for 20 minutes. The 2 ml of transfection mixture was added to the flask of cells containing 8 ml of medium and the resulting mixture was left in a CO₂ incubator overnight before removing the transfected cells and adding to the 96-well plate containing the immobilised Notch ligand protein.

The following day the transfected CHO-N2 cells were removed using 0.02% EDTA solution (Sigma), spun down and resuspended in 10 ml DMEM plus 10%(HI)FCS plus glutamine plus P/S. 10 µl of cells were counted and the cell density was adjusted to 2.0 x 10⁵ cells/ml with fresh DMEM plus 10%(HT)FCS plus glutamine plus P/S. 100 µl per well was added to a 96-well tissue culture plate (flat bottom), i.e. 2.0 x 10⁴ transfected cells per well, using a multi-channel pipette and the plate was then incubated overnight.

### D)Immobilisation of Notch Ligand protein directly onto a 96-well Tissue Culture Plate

10 µg of purified Notch ligand protein was added to sterile PBS in a sterile Eppendorf tube to give a final volume of 1 ml. Serial 1:2 dilutions were made by adding 500 µl into sterile Eppendorf tubes containing 500 µl of sterile PBS to generate dilutions of 10 µg/ml, 5 µg/ml, 2.5 µg/ml, 1.25 µg/ml, 0.625 µg/ml and 0 µg/ml.

The lid of the plate was sealed with parafilm and the plate was left at 4 °C overnight or at 37°C for 2 hours. The protein was then removed and the plate was washed with 200 µl of PBS.

### E) A2O-Delta cells

The IVS, IRES, Neo and pA elements were removed from plasmid pIRESneo2 (Clontech, USA) and inserted into a pUC cloning vector downstream of a chicken beta-actin promoter (eg see GenBank Accession No E02199). Mouse Delta-1 (eg see GenBank Accession No NM_007865) was inserted between the actin promoter and IVS elements and a sequence with multiple stop codons in all three reading frames was inserted between the Delta and IVS elements.

The resulting construct was transfected into A20 cells using electroporation and G418 to provide A20 cells expressing mouse Delta1 on their surfaces (A20-Delta).

### F) CHO and CHO-hDelta1-V5-His Assay Control

CHO cells were maintained in DMEM plus 10%(HI)FCS plus glutamine plus PIS and CHO-hDelta1-V5-His (clone#10) cells were maintained in DMEM plus 10%(HI)FCS plus glutamine plus P/S plus 0.5mg/ml G418.

Cells were removed using 0.02% EDTA solution (Sigma), spun down and resuspended in 10 ml DMEM plus 10%(HI)FCS plus glutamine plus P/S. 10 µl of cells were counted and the cell density was adjusted to 5.0 x 10⁵ cells/ml with fresh DMEM plus 10%(HI)FCS plus glutamine plus P/S. 300 µl of each cell line at 5.0 x 10⁵ cells/ml was added into duplicate wells of a 96-well tissue culture plate. 150 µl of DMEM plus 10%(HI)FCS plus glutamine plus P/S was added in to the next 5 wells below each well. 150 µl of cells were serially diluted into the next 4 wells giving cell density dilution of 5.0 x 10⁵ cells/ml, 2.5 x 10⁵ cells/ml, 1.25 x 10⁵ cells/ml, 0.625 x 10⁵ cells/ml, 03125 x 10⁵ cells/ml and 0 cells/ml.

100 µl from each well was added into the 96-well plate containing 100 µl of CHO-N2 cells transfected with 10xCBF1-Luc (2.0 x10⁴ transfected CHO-N2 cells/well) and the plate was left in an incubator overnight.

### G) Cell Co-Culture

5 x 10⁴ CHO-N2 cells were plated on a 96 well plate. CHO-Delta or A20-Delta cells were titrated in as required (max ratio CHO-N2: CHO-Delta was 1:1, max ratio CHO-N2: A20-Delta was 1:2). The mixture was incubated overnight before conducting a luciferase assay.

### H) Luciferase Assay

Supernatant was removed from all wells. 100 µl of PBS and 100 µl of SteadyGlo^{™} luciferase assay reagent (Promega) was added and the cells were left at room temperature for 5 minutes. The mixture was pipetted up and down 2 times to ensure cell lysis and contents from each well were transferred into a white 96-well OptiPlate^{™} (Packard). Luminescence was measured in a TopCount^{™} counter (Packard).

All publications mentioned in the above specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the following claims.

### References incorporated herein by reference thereto)

Hoyne, G. et al. (2000) Immunology 100:281-288.
Lu, F. M. et al. (1996) Proc Natl Acad Sci 93(11):5663-7.
Lieber, T. et al. (1993) Genes Dev 7(10):1949-65.
Struhl, G. et al. (1998) Cell 93(4):649-60.
Matsuno, K. et al. (1995) Development 121(8):2633-44.
Schroeter, E.H. et al. (1998) Nature 393(6683):382-6.
Jarriault S. et al. (1995) Nature 377(6547): 355-8.
Takebayashi K. et al. (1994) J Biol Chem 269(7):150-6.
Leimeister C. et al. (1999) Mech Dev 85(1-2):173-7.
Chee M. et al. (1996) Science 274:601-614.
Camilli et al. (1994) Proc Natl Acad Sci USA 91:2634-2638.
Pasqualini R, Ruoslahti E. (1996) Nature 380:364-366.
Ruoslahti (1996) E. Ann. Rev. Cell Dev. Biol. 12:697-715.
Arap, W, Pasqualini, R, Ruoslahti, E (1998) Science 279:377-380.
Ordentlich et al. (1998) Mol. Cell. Biol. 18:2230-2239.
Tamura K, et al. (1995) Curr. BioL 5:1416-1423.
Artavanis-Tsakonas S, et al. (1995) Science 268:225-232.
Munro S, Freeman M. (2000) Curr. BioL 10:813-820.
Ju BJ, et al. (2000) Nature 405:191-195.
Moloney DJ, et al. (2000) Nature 406:369-375.
Brucker K, et al. (2000) Nature 406:411-415.
Panin VM, et al. (1997) Nature 387:908-912.
Hicks C, et al. (2000) Nat. Cell. Biol. 2:515-520.
Irvine KD (1999) Curr. Opin. Genet. Devel. 9:434-441.
Schroeter EH, et al. (1998) Nature 393:382-386.
Struhl G, Adachi A. (1998) Cell 93:649-660.
Weinmaster G. (2000) Curr. Opin. Genet. Dev. 10:363-369.
Artavanis-Tsakonas S, et al. (1999) Science 284:770-776.
Osbome B, Miele L. (1999) Immunity 11:653-663.
Matsuno K, et al. (1998) Nat. Genet. 19:74-78.
Medhzhitov et al. (1997) Nature 388:394-397.
Li et aL (1998) Immunity 8(1):43-55.
Iemura et al. (1998) PNAS 95:9337-9345.
Valenzuela et al. (1995) J. Neurosci. 15:6077-6084.
Sasai et al. (1994) Cell 79:779-790.
Wilson and Hemmati-Brivanlou (1997) Neuron 18:699-710.
Hemmati-Brivanlou and Melton (1997) Cell 88:13-17.
Dkuz et al. (1997) Cell 90:271-280.
Zhao et al. (1995) J. Immunol. 155:3904-3911.
Kum et al. (2000) Can J Microbiol 46(2):171-9.
Rubinchik & Chow (2000) Vaccine 18(21):2312-20.
Wahlsten & Ramakrishnan (1998) J. Immunology 160:854-859.
Papageorgiou et aL (1999) The EMBO Journal 18(1):9-21.
Roussel et al (1997) Nat Struct Biol 4(8) :635-43.
Sundstrom et al. (1996) EMBO J 15(24):6832-40.

Various preferred features and embodiments of the present invention will now be described with reference to the following numbered paragraphs (paras).
1. A conjugate comprising first and second sequences wherein the first sequence comprises a polypeptide which is capable of binding to an antigen presenting cell (APC) or a polynucleotide encoding therefor, and the second sequence comprises a polypeptide capable of modulating of a T cell signalling pathway, or a polynucleotide encoding therefor.
2. A conjugate according to para 1 in the form of a fusion protein.
3. A conjugate according to para 1 or para2 wherein the second sequence is a protein for T cell receptor signalling transduction or a polynucleotide sequence encoding said protein for T cell receptor signalling transduction.
4. A conjugate according to para 3 wherein the second sequence is a protein for activation of a T cell costimulatory molecule or a polynucleotide encoding therefor.
5. A conjugate according to any preceding para wherein the second sequence is a protein for Notch signalling transduction or a polynucleotide sequence encoding said protein for Notch signalling transduction.
6. A conjugate according to para 5 wherein the second sequence is Notch or a fragment thereof which retains the signalling transduction ability of Notch or an analogue of Notch which has the signalling transduction ability of Notch, or a polynucleotide sequence which encodes therefor.
7. A conjugate according to para 5 wherein the second sequence is a Notch ligand or a fragment thereof which retains the signalling transduction ability of Notch ligand or an analogue of Notch ligand which has the signalling transduction ability of Notch ligand, or a polynucleotide sequence which encodes therefor.
8. A conjugate according to para 7 wherein the second sequence is derived fromDelta or Serrate, or a polynucleotide sequence which encodes therefor.
9. A conjugate according to para 5 wherein the second sequence is a polypeptide capable of upregulating the expression or activity of Notch, a Notch ligand or a downstream component of the Notch signalling pathway, an antibody or a polynucleotide which encodes therefor.
10. A conjugate according to para 9 wherein the second sequence is selected from Noggin, Chordin, Follistatin, Xnr3, fibroblast growth factors, immunosuppressive cytokines and derivatives, fragments, variants and homologues thereof, or a polynucleotide which encodes therefor.
11. A conjugate according to para 10 wherein the second sequence is an immunosuppressive cytokine selected from IL-4, IL-10, IL-13, TGF-β and SLIP3 ligand, or a polynucleotide which encodes therefor.
12. A conjugate according to para 5 wherein the second sequence is a protein for Notch signalling inhibition or a polynucleotide sequence encoding said protein for Notch signalling inhibition.
13. A conjugate according to para 12 wherein the second sequence is a polypeptide capable of downregulating the expression or activity of Notch, a Notch ligand or a downstream component of the Notch signalling pathway, an antibody or a polynucleotide which encodes therefor.
14. A conjugate according to para 13 wherein the second sequence is selected from Toll-like receptors (TLRs), cytokines, bone morphogenic proteins (BMPs), BMP receptors, activins and derivatives, fragments, variants and homologues thereof, or a polynucleotide which encodes therefor.
15. A conjugate according to para 14 wherein the second sequence is a cytokine selected from IL-12, TFN-(and TFN-∀, or a polynucleotide which encodes therefor.
16. A conjugate according to any preceding para wherein the first sequence is a polypeptide which is capable of binding to an APC surface molecule, or, is a polynucleotide which encodes therefor.
17. A conjugate according to para 16 wherein the APC surface molecule is an MHC class II molecule, CD205 (DEC205), CD204 (Scavenger receptor), CD14, CD206 (Mannose receptor), TLRs, Langerin (CD207), DC-SIGN (CD209), Fcγ receptor 1 (CD64), Fcγ receptor 2 (CD32), CD68, CD83, CD33, CD54 or BDCA-2,3,4.
18. A conjugate according to any preceding para wherein the first sequence comprises a polypeptide which is capable of binding to an MHC class II molecule.
19. A conjugate according to any preceding para wherein the first sequence is or is derived from a superantigen.
20. A conjugate according to any preceding para wherein the first sequence is or is derived from bacterial or viral superantigen.
21. A conjugate according to para 19 or 20 wherein the first sequence comprises the MHC class II binding domain of a superantigen.
22. A conjugate according to any one of paras 19 to 21 wherein the superantigen is selected from the group of staphylococcal enterotoxins (SEs), such as SEA, SEB, SEC, SED, SEE and SEH.
23. A conjugate according to para 21 wherein the superantigen is TSST-1.
24. A conjugate according to any one of paras 19 to 21 wherein the superantigen is selected from Streptococcal enterotoxins (SPE), such as SPEA, SPEC and SSA.
25. A polynucleotide sequence encoding the conjugate of any preceding para.
26. An expression vector comprising the polynucleotide sequence of para 25.
27. A host cell transformed with the expression vector of para 26.
28. A method for preparing a conjugate comprising culturing the host cell of para 27 under conditions which provide for the expression of the conjugate.
29. A conjugate prepared by the method of para 28.
30. A method of targeting a protein for Notch signalling modulation or a polynucleotide sequence which encodes therefor to an APC comprising exposing an APC to a conjugate according to any one of paras 1 to 24 or 29.
31. A pharmaceutical composition comprising the conjugate of any of paras 1 to 24 or 29 and a pharmaceutically acceptable excipient, diluent or carrier.
32. A pharmaceutical composition according to para 31 for use in the treatment of T-cell mediated disease.
33. Use of the conjugate of any of paras 1 to 24 or 29 in the preparation of a medicament for the prevention and/or treatment of disease or infection.
34. Use according to para 33 wherein the disease is a T-cell mediated disease.
35. A conjugate, polynucleotide sequence, expression vector, host cell, method, pharmaceutical composition or use substantially as hereinbefore described with reference to the accompanying Figures.

## Claims

1. A conjugate comprising first and second sequences wherein the first sequence is an antibody or antibody fragment which is capable of binding to an APC surface molecule, or a polynucleotide encoding therefor, and the second sequence comprises a polypeptide for Notch signalling transduction or a polynucleotide sequence encoding said polypeptide for Notch signalling transduction.

2. A conjugate according to claim 1 in the form of a fusion protein.

3. A conjugate according to claim I wherein the second sequence comprises a Notch ligand DSL domain and at least one EGF domain.

4. A conjugate according to any preceding claim wherein the second sequence comprises a Notch ligand DSL domain and at least 3 to 8 Notch ligand EGF repeat motifs.

5. A conjugate according to any preceding claim wherein the second sequence is a Notch ligand or a fragment thereof which retains the signalling transduction ability of Notch ligand, or a polynucleotide sequence which encodes therefor.

6. A conjugate according to any preceding claim wherein the second sequence comprises a human Notch ligand sequence.

7. A conjugate according to any preceding claim wherein the second sequence is derived from Delta or Serrate, or a polynucleotide sequence which encodes therefor.

8. A conjugate according to any preceding claim wherein the APC surface molecule is an MHC class II molecule, CD205 (DEC205), CD204 (Scavenger receptor), CD14, CD206 (Mannose receptor), TLRs, Langerin (CD207), DC-SIGN (CD209), Fcγ receptor 1 (CD64), Fcγ receptor 2 (CD32), CD68, CD83, CD33, CD54 or BDCA-2,3,4.

9. A conjugate according to any preceding claim wherein the first sequence is an antibody or antibody fragment which is capable of binding to an MHC class II molecule.

10. A method of targeting a protein for Notch signalling modulation or a polynucleotide sequence which encodes therefor to an APC comprising exposing an APC to a conjugate according to any one of claims 1 to 9.

11. A pharmaceutical composition comprising the conjugate of any of claims 1 to 9 and a pharmaceutically acceptable excipient, diluent or carrier.

12. A pharmaceutical composition according to claim 11 for use in the treatment of T-cell mediated disease.

13. Use of the conjugate of any of claims 1 to 9 in the preparation of a medicament for the prevention and/or treatment of disease or infection.

14. Use according to claim 13 wherein the disease is a T-cell mediated disease.

15. Use according to claim 14 wherein the disease is asthma, allergy, graft rejection autoimmunity, tumour induced aberrations to the T cell system or infectious disease.
